# EUROPEAN PATENT SPECIFICATION

(11) **EP 2 230 242 B1**
(45) Date of publication and mention of the grant of the patent: **14.08.2013**
(21) Application number: 09003727.6
(22) Date of filing: 16.03.2009
(51) Int. Cl.: C07D 487/22, A61K 31/4425, A61K 31/555, A61P 35/00

(54) **Synthesis of new asymmetrically meso-substituted water-soluble porphyrins acting as cytostatic agents**
Synthese von neuen asymmetrisch mesosubstituierten wasserlöslichen, als zytostatische Mittel wirkenden Porphyrinen
Synthèse des nouvelles porphyrines solubles dans l'eau méso-substituées agissant en tant qu'agents cytostatiques

(43) Date of publication of application: 22.09.2010
(73) Proprietor: Johannes Kepler Universität, 4040 Linz (AT)
(72) Inventor: Häubl, Martin, 4040 Linz (AT); Schöfberger, Wolfgang, 4040 Linz (AT); Schürz, Susanne, 4063 Hörsching (AT); Müller, Norbert, 4210 Gallneukirchen (AT); Svejda, Bernhard, 9640 Kschach-Mauthen (AT); Pfragner, Roswitha, 8051 Graz (AT)
(74) Representative: Landgraf, Elvira

(56) References cited:
- WO-A-91/10667
- WO-A-98/33503
- BEDEL-CLOUTOUR C H ET AL: "SYNTHESES OF FUNCTIONALIZED INDIUM PORPHYRINS FOR MONOCLONAL ANTIBODY LABELING" BIOCONJUGATE CHEMISTRY, ACS, WASHINGTON, DC, US, vol. 7, no. 6, 1 November 1996 (1996-11-01), pages 617-627, XP000638680 ISSN: 1043-1802
- TREMBLAY-MORIN J P ET AL: "First example of a palladium catalyzed coupling reaction between cationic porphyrins and alkynyls in aqueous medium" TETRAHEDRON LETTERS, ELSEVIER, AMSTERDAM, vol. 46, no. 41, 10 October 2005 (2005-10-10), pages 6999-7002, XP025385017 ISSN: 0040-4039 [retrieved on 2005-10-10]
- SUGIMOTO NAOKI ET AL: "Interactions of 5-(1-pyrenyl)-10,15,20-tris(4-methylpyridi nium)porph ine with double-helix and triple-helix nucleic acids" NUCLEOSIDES & NUCLEOTIDES, MARCEL DEKKER, INC, US, vol. 15, no. 1/3, 1 January 1996 (1996-01-01), pages 743-747, XP009119758 ISSN: 0732-8311

## Description

### Field of the invention

The present invention relates to substituted watersoluble cationic porphyrin compounds and novel cationic gold(III)porphyrins, a process for preparing said compounds, to pharmaceutical compositions comprising said compounds and their use, especially as anti-tumor agents.

### Background of the invention

Cationic porphyrinoid systems are used in various fields of oncology. Due to their ability to act as photosensitizing agents porphyrins can be used in photodyamic therapy, where they are efficient singlet oxygen producers (Juarranz, A., Jaén, P., Sanz-Rodrigez, F., Cuevas, J., and González, S. (2008). Photodynamic therapy of cancer. Basic principles and applications. Clin. Transl. Oncol. 10, 148-154, Dolmans, D.E.J.G.J., Fukumura, D., and Jain, R.K. (2003). Photodynamic therapy for cancer. Nature Reviews Cancer 3, 380-387, Brown, S.B., Brown, E.A., and Walker, I. (2004). The present and future role of photodynamic therapy in cancer treatment. Oncology 5, 497-508.)

Next to that these compounds are able to intercalate in duplex- (Ohyama, T., Mita, H., and Yamamoto, Y. (2005). Binding of 5,10,15,20-tetrakis(N-methylpyridinium-4-yl)-21 H,23H-porphyrin to an AT-Rich Region of a Duplex DNA. Biophysical Chemistry 113, 53-59, Guliaev, A.B., and Leontis, N.B. (1999). Cationic 5,10,15,20-Tetrakis(N-methylpyridinium-4-yl)porphyrin Fully Intercalates at 5'-CG-3' Steps of Duplex DNA in Solution. Biochemistry 38, 15425-15437).and in quadruplex DNA Weelhouse, R.T., Sun, D., Han, H., Han, F.X., and Hurley, L.H. (1998). Cationic porphyrins as telomerase inhibitors: the interaction of tetra(N-methyl-4-pyridyl)porphine with quadruplex DNA J. Am. Chem. Soc. 120, 3161-3162, Shi, D.-F., Wheelhouse, R.T., Sun, D., and Hurley, L.H. (2001). Quadruplex-Interactive Agents as Telomerase Inhibitors: Synthesis of Porphyrins and Structure-Activity Relationship for the Inhibition of Telomerase J. Med. Chem. 44, 4509-4523.This may have an effect on the replication and transcription process next to telomere length due to stabilization of duplex DNA or quadruplex DNA. Intercalation in duplex DNA can also lead to frameshift mutations. All these effects significantly inhibit cell growth or even induce cell death. Recently some research groups supposed that small ligands like porphyrins are able to interact with the p53 tumor suppressing protein regulatory mechanism (Zawacka-Pankau, J., Krachulec, J., Grulkowski, I., Bielawski, K.P., and Selivanova, G. (2008). The p53-mediated cytotoxicity of photodynamic therapy of cancer: Recent advances Toxicology and Applied Pharmacology 232, 487-497).

Gold (III) porphyrins have received enormous interest e.g. as potential anticancer drugs (Wang Y., He Q.-Y., Che C.-M. and Chiu J.-F., Proteomic characterization of the cytotoxic mechanism of gold (III) porphyrin 1a, a potential anticancer drug Proteomics, 2006, 6, 131-142,Wang Y., He Q.-Y., Sun R. W.-Y., Che C.-M. and Chiu J.-F., Gold(III) Porphyrin 1a Induced Apoptosis by Mitochondrial Death Pathways Related to Reactive Oxygen Species Cancer Res., 2005, 65, 11553-11564, WO 2004/024146.

Being isostructural and isoelectronic to platinum (II), the gold (III) ion forms square planar complexes and, therefore, has long been anticipated to be a promising anti-tumor agent. To date, two major classes of anti-tumor gold (III) complexes have been extensively studied Mirabelli, C.K. & co-workers Interactions of gold coordination complexes with DNA Biochem. Pharm. 1986, 35, 1427, Dabrowiak, J.C. & co-workers, DNA binding specificity of the goid(III) complex (C2H5)3PAuBr3, J. Am. Chem. Soc. 1987, 109, 3810, Parish, R.V. & co-workers, Chemical and Biological Studies of Dichloro(2-((dimethylamino)methyl)phenyl)gold(III) Inorg. Chem. 1996, 35, 1659, Orioli, P. & co-workers, Structure and DNA binding properties of the gold(III) complex [AuCl2(esal)], Inorg. Chim. Acta 1999, 285, 309, Orioli, P. & co-workers, Gold(III) compounds as potential antitumor agents: Cytotoxicity and DNA binding properties of some selected polyamine-gold(III) complexes, Inorg. Chim. Acta, 1998, 281, 90, Orioli, P. & co-workers, Cytotoxicity and DNA binding properties of a chloro glycylhistidinate gold (III) complex, Chem. Biol. Interact. 2000, 125, 29, Orioli, P. & co-workers, Gold(III) Complexes as Potential Antitumor Agents: Solution Chemistry and Cytotoxic Properties of Some Selected Gold(III) Compounds, J. Med. Chem. 2000, 43, 3541, Orioli, P. & co-workeres Gold(III) complexes as a new family of cytotoxic and antitumor agents, Expert Review of Anticancer Therapy, Vol. 2, Number 3, june 2002 (10), 337-346, Calamai, P., Carotti, S., Guerri, A., Messori, L., Mini, E., Orioli, P., Speroni G., Biological properties of two gold(III) complexes: AuCl3 (Hpm) and AuCl2 (pm), Journal of Inorganic BiochemistryVol. 66, Issue 2, 1997, 103-109, Marcon, G., Carotti, S., Coronnello, M., Messori, L., Mini, E., Orioli, P., Mazzei, T., Cinellu, M. A., Minghetti, G., Gold(III) Complexes with Bipyridyl Ligands: Solution Chemistry, Cytotoxicity, and DNA Binding Properties, J.Med. Chem. 2002 45 (8), 1672-1677.

There are compounds which encompass those gold (III) complexes containing one, two or three labile ligand(s) (e.g., -halide, -SCN) and a mono-/bi-/tridentate auxiliary ligand and compounds, where the four-coordinated gold(III) atom without labile ligand(s) is coordinated to some chelating polyamine ligands. According to the literature, all these gold (III) compounds have limited stability in physiological buffer solutions; especially they are readily reduced by mild reductants such as sodium thiosulfate and glutathione to give colloidal gold. To our knowledge, there has been limited success in preparing potent anti-tumor gold (III) compounds that are stable in physiologically relevant conditions. Therefore we have synthesized a new cytotoxic photosensitizer, which combines the high DNA binding affinity of cationic polycyclic aromatic hydrocarbons with the pro-drug properties of gold-based chemotherapeutic agents.

WO 98/33503 A discloses water soluble meso-substituted porphyrins for use in the treatment of cancer.

Sugimoto Naoki et al. "Interactions of 5-(1pyrenyl)-10.15.20-tris(4-methylpyridinium)porphyrine with double helix and triple helix nucleic acids", Nucleosis & Nucleotides, vol 15, 1996, pages 743-747 discloses poryhrins with a pyrenyl substituent in the 20-position as potential anti cancer agents.

### Medical applications of porphyrins in oncology

(Haemato)-porphyrin derivatives of uncertain specific composition have been used in cancer treatment, having found to locate in tumors and other tissues after injection into the bloodstream and to sensitize cells to light irradiation. Irradiation may be carried out directly or indirectly with a laser or other source. Irradiated cells are rapidly destroyed to a depth depending on the depth of light penetration.

US 4 386 087 A discloses sulfonated tetraphenylporphyrins for treatment of leukemia.

EP 0 066 884 discloses iron complexes of substituted tetra-amino porphyrins for use as oxygen absorbing agents.

CA 1 257 202 A discloses substituted meso porphyrins for use in pharmaceutical agents for locating tumors, which are then irradiated with a light source.

US 5,986,090 discloses porphyrin derivatives and method for producing substituted porphyrin compounds and polymers containing the same.

US 6,953,570 discloses a method for increasing the multi photon absorption cross section of a porphyrin based photosensitizer.

WO 2004/24146 discloses gold(III) prophyrin derivatives as anti tumor and anti HIV agents.

Unfortunately most of the known porphyrin compounds useful in treatment of cancer do not only locate and selectively destroy tumor cells, but especially upon exposure to light also harm adjacent normal cells.

### Object of the invention

It was an object of the invention to provide metalated and non-metalated substituted cationic porphyrin compounds which selectively locate in tumor cells and selectively destroy tumor cells, without harming adjacent normal cells. Further it was an object of the invention to provide a process for producing such substituted cationic porphyrins, as well as pharmaceutical agents made therefrom.

It was the object if the invention to provide substituted porphyrin compounds which selectively locate in tumor cells and selectively destroy tumor cells, without harming adjacent normal cells. Further it was an object of the invention to provide a process for producing such substituted porphyrins, as well as pharmaceutical agents made therefrom. First studies in anticancer drug screening were done in two types of tumor cell cultures. We selected cell lines of a medullary thyroid carcinoma, MTC-SK (Pfragner, R., Hoefler, H., Behmel, A., Ingolic, E., Walser, V. (1990). Establishment and characterization of continuous cell line MTC-SK derived from a human medullary thyroid carcinoma. Cancer Res. 50, 4160-4166.) and a carcinoid of the small intestine, KRJ-I (Pfragner, R., Wirnsberger, G., Niederle B., Behmel, A., Rinner I., Mandl, A., Wawrina, F., Luo. J. S., Adamiker, D., Hoeger, H., Ingolic, E. and Schauenstein, K. (1996) Establishment of a continuous cell line from a human carcinoid of the small intestine (KRJ-I): characterization and effects of 5-azacytidine on proliferation. Int. J. Oncol. 8, 513-520.). Both neuroendocrine tumors are known for their poor response to chemotherapy or radiotherapy. Cell lines derived from these tumors were treated with porphyrins and analyzed by different methods: Growth kinetics were examined by using the Casy-1 Cell Counter & Analyzer (Schärfe, Germany), by a Cell Counting. Cell numbers and the quantity of dead cells were determined with a Casy-1 Cell Counter & Analyzer Model TTC (Schärfe System, Reutlingen, Germany, Cell Proliferation Reagent WST-1 Roche Diagnostics, Vienna, Austria). Cell proliferation and viability were quantified using the WST-1 cell proliferation reagent (4-[3-(4-iodophenyl)-2-(4-nitrophenyl)-2H-5-tetrazolio]--1, 3-benzene disulphonate) This method is based on the ability of viable cells to metabolize tetrazolium salt WST-1 to formazan by mitochonrial dehydrogenases.

CytoTox-GLO Cytotoxicity Assay. The CytoTox-GLO Cytotoxicity Assay allows the selective measurement of the number of dead cells on cell populations. The test measures a distinct protease activity associated with cytotoxicity, using a luminogenic peptide substrate (alanyl-alanyl-phenylalanyl-aminoluciferin, AAF-Glo Substrate) to measure dead cell protease activity which has been released from cells that have lost membrane integrity.

### Brief description of the invention

The invention relates to substituted porphyrin compounds of formula 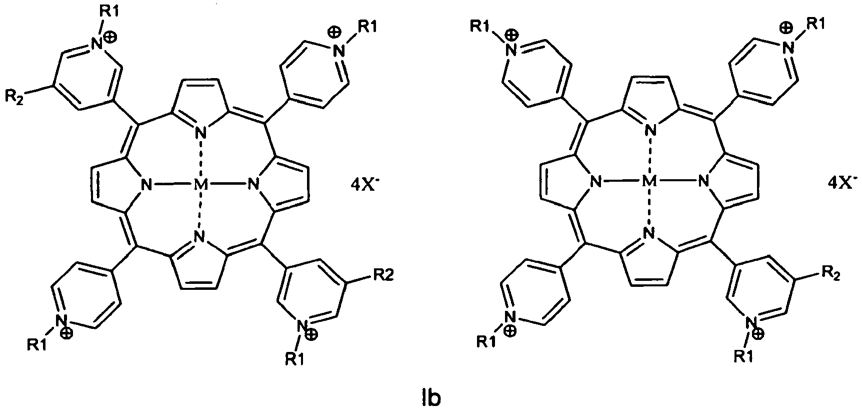 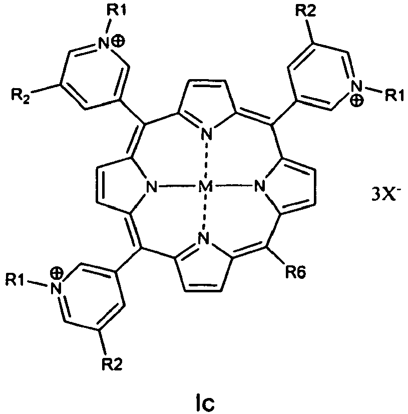 in which R1 is a C₁ to C₄ alkyl group, R2 is halogen or an aryl or heteroaryl group, which may be substituted by groups shown in formula II 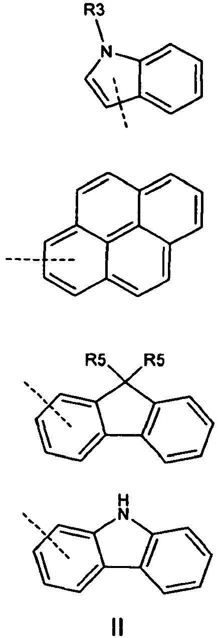 in which R3 is H or a protecting group such as a group SO₂R4, in R4 is a aryl or an heteroaryl group R5 is a H or a C₁ to C₄ alkyl group, R6 are the substituents shown in formula III 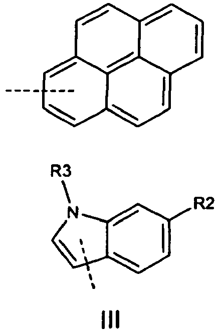 X is a pharmaceutical acceptable counter ion, M is a transition metal ion and pharmaceutically acceptable salts and metal complexes thereof.

In formula I R1 denotes a C₁ to C₄ alkyl group, for example a methyl, ethyl, propyl or butyl group.

R2 denotes halogens, preferably Cl, Br, I or an aryl or heteroaryl group. Aryl is preferably taken to mean C₆-C₂₀-aryl groups, for instance phenyl, biphenyl, naphthyl, indenyl, fluorenyl etc.

Heteroaryl- or heterocycle is taken to mean cyclic radicals which contain at least one O, S or N atom in the ring. These are, for example, furyl, thiophenyl, pyridyl, pyrimidyl, imidazolyl, tetrazolyl, pyrazinyl, benzofuranyl, quinolyl, isoquinolyl, isobenzofuryl, pyrazolyl, indolyl, isoindolyl, benzoimidazolyl, purinyl, carbazolyl, oxazolyl, isoxazolyl, pyrrolyl, quinazolinyl, pyridazinyl, phthalazinyl, morpholinyl etc. Functional O or N groups can be protected here if necessary. These groups can be monosubstituted by an indole group. The indole group is preferably substituted by a group R3 which is defined above.

The chromophores of formula Ia and Ib can be prepared by methods known by the art, for example by acid catalyzed condensation of various aldehydes or by the "Mac Donald 2+2 method for synthesizing porphyrins from dipyrromethanes (Arsenault et al. J. Chem. Soc. 1960, 82: 4384 - 4389).

These compounds are further modified by Suzuki coupling reactions and are transformed into water-soluble species by N-alkylation. The obtained salts are converted to chlorides by an ion exchange resin.

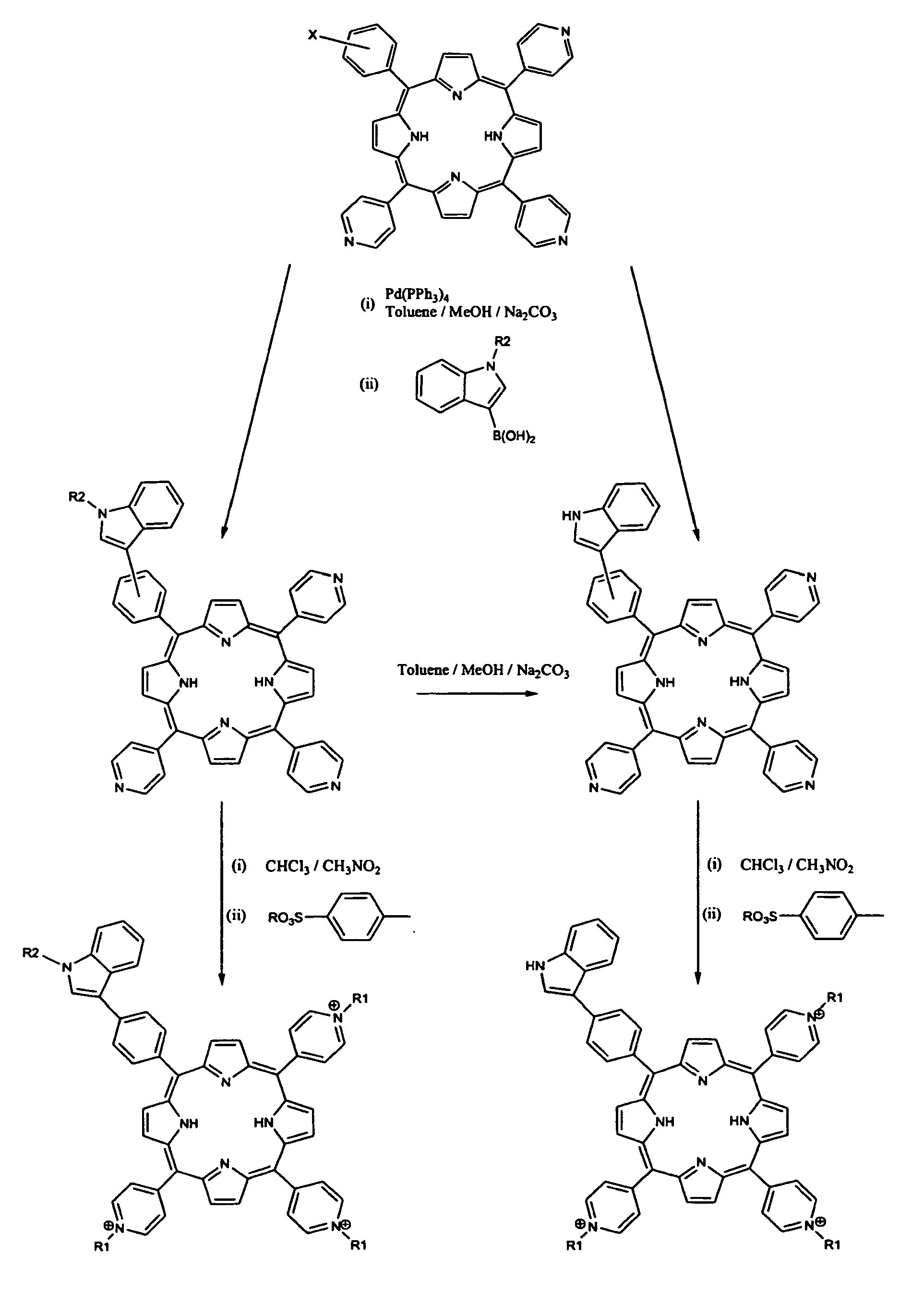

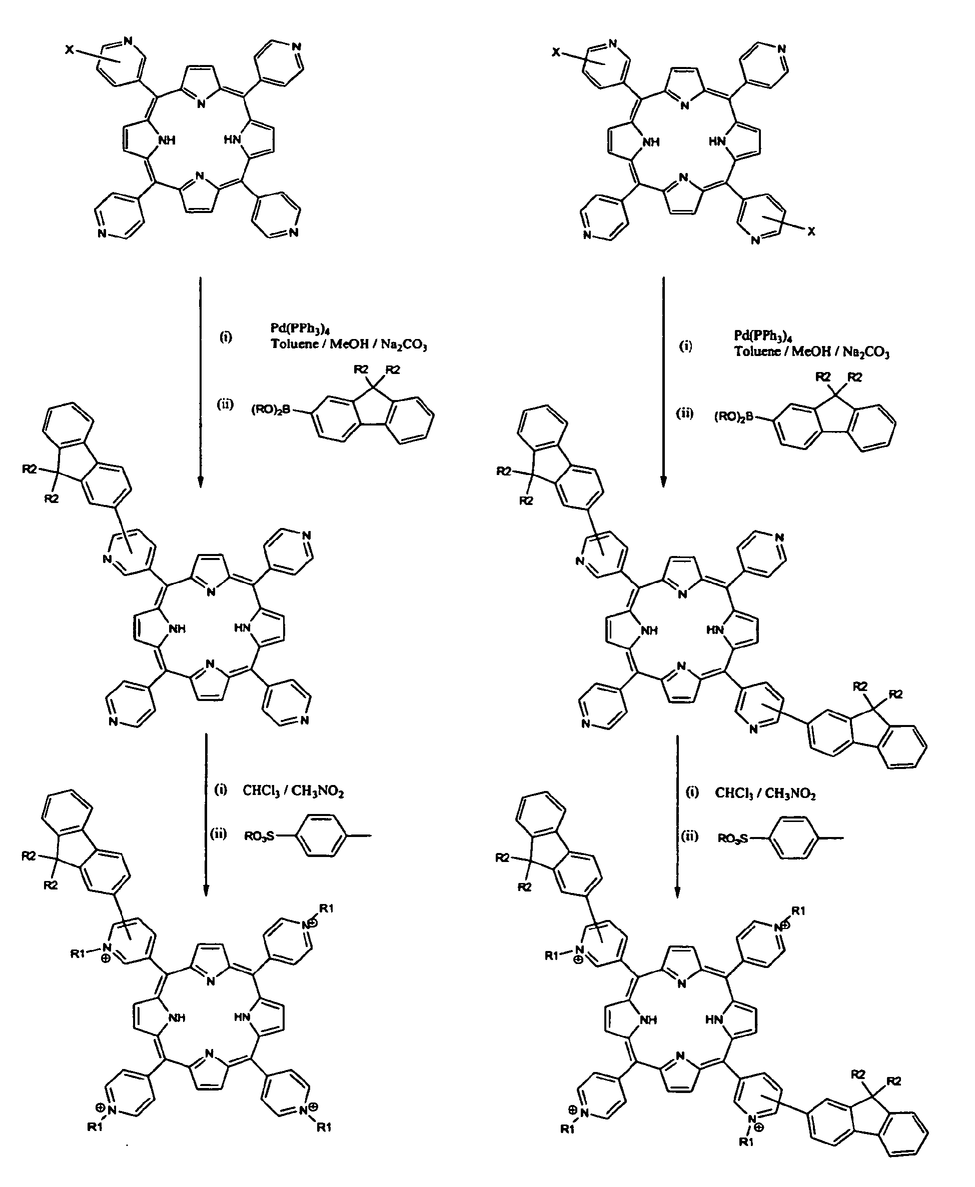

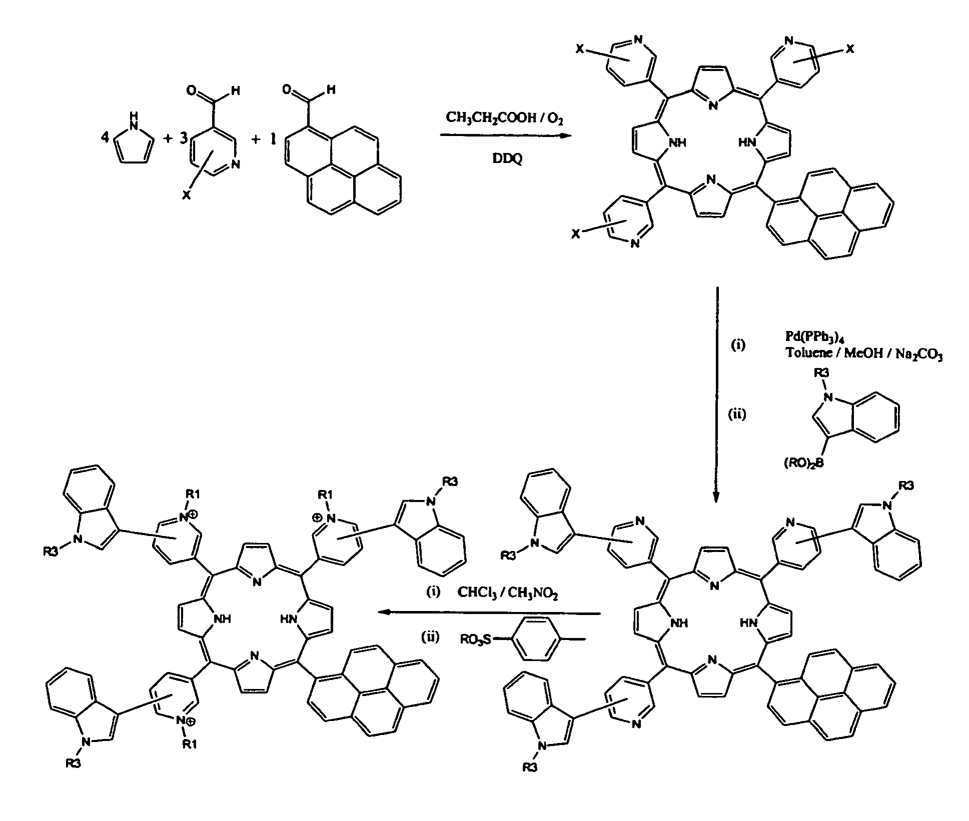

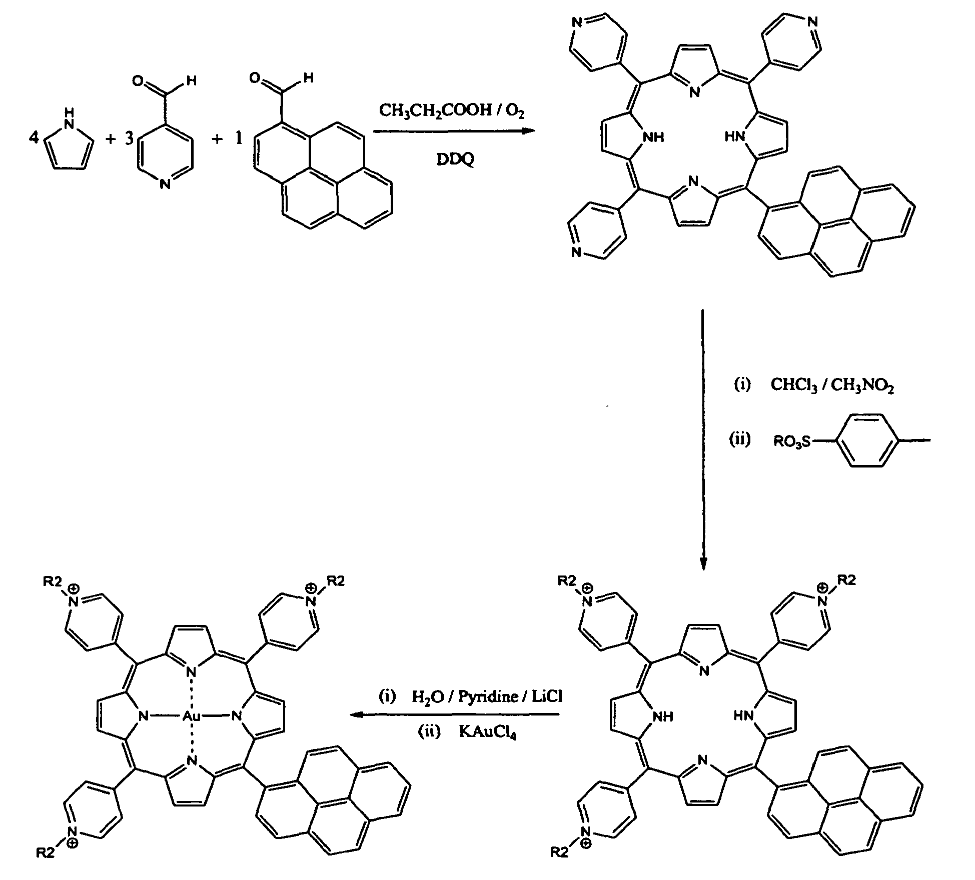

The compounds of formulas Ia, Ib, Ic respectively their pharmaceutically acceptable salts may be used for the preparation of pharmaceuticals useful for treating cancer, especially thyroid cancer.

### Cancer cell line tests

The synthesized compounds have been tested on two tumor cell lines. KRJ-I, which is a carcinoid cell line from the small intestine and MTC-SK, which is a medullary thyroid carcinoma cell line. The effect on those is compared with that on HF-SAR fibroblast cells. This is an adherent human cell line, which represent normal tissues. For these tests the following methods have been used: WST1 Cell Proliferation Assay, Casy1 Cellcounter and a cytotoxicity assays.

### Comparison of the cytostatic activity of porphyrin compounds on KRJ-I, MTC-SK and HF-SAR

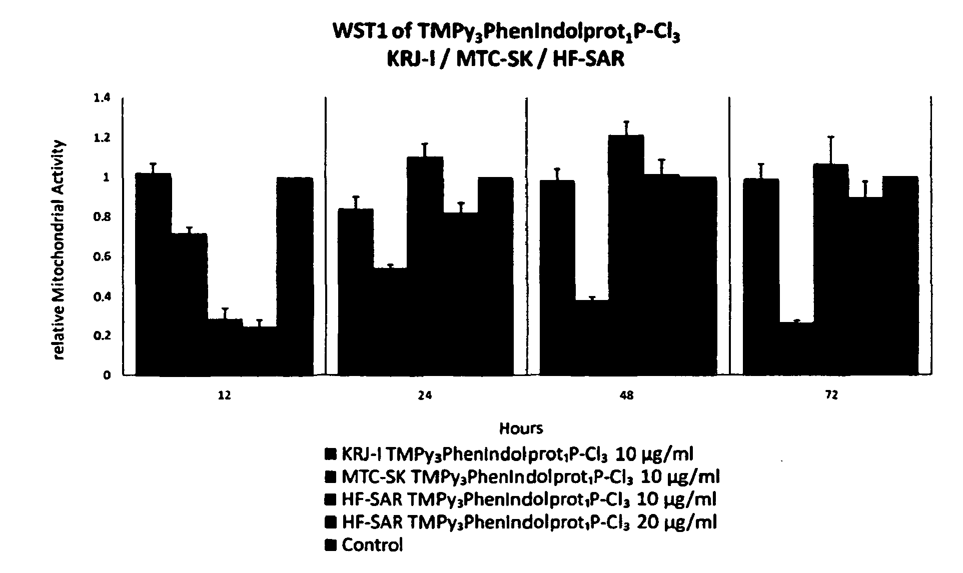

Comparison of the mitochondrial activity of MTC-SK, KRJ-I and HF-SAR cells.

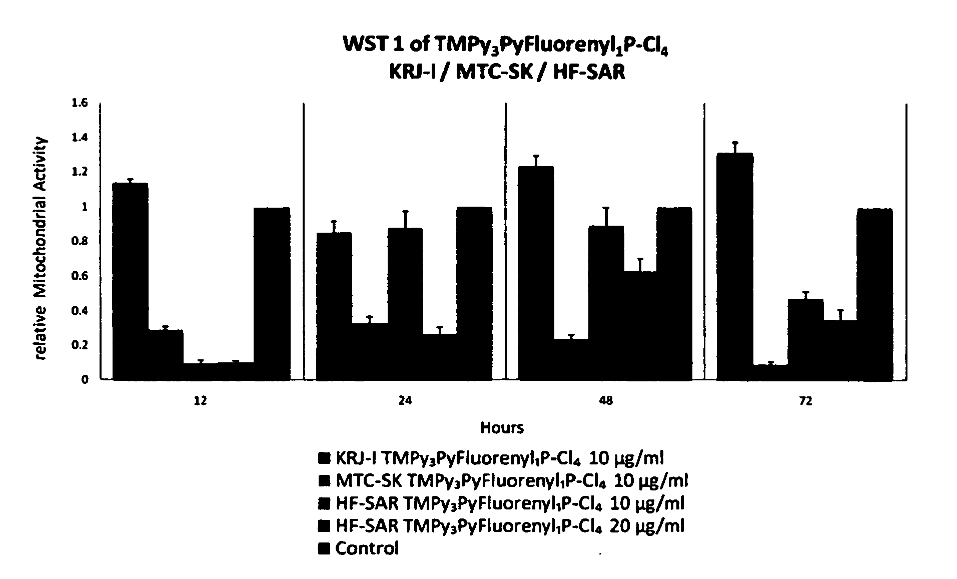

Comparison of the mitochondrial activity of MTC-SK, KRJ-I and HF-SAR cells. TMPy₃PhenIndolprot₁P-Cl₃ exhibits a strong inhibitory effect on the growth of MTC-SK cancer cells. Additionally a weak inhibition of the growth of KRJ-I cancer cells is observed as well. Although this substance reduces the mitochondrial activity of the HF-SAR fibroblast cells (normal cells) within the first 12 h, the mitochondrial activity is restored after 24 h and reaches a level similar to the control group. In comparison to that, the mitochondrial activity of MTC-SK tumor cells is decreasing steadily during incubation time. Therefore TMPy₃Phenlndolprof₁P-Cl₃ selectively induces cell growth inhibition to MTC-SK tumor cells, while normal cells as the HF-SAR fibroblasts are hardly affected.

### Cytotoxicity, Casy1 Cell counts and WST-1 mitochondrial activity test of KRJ-I tumor cells upon incubation with porphyrins

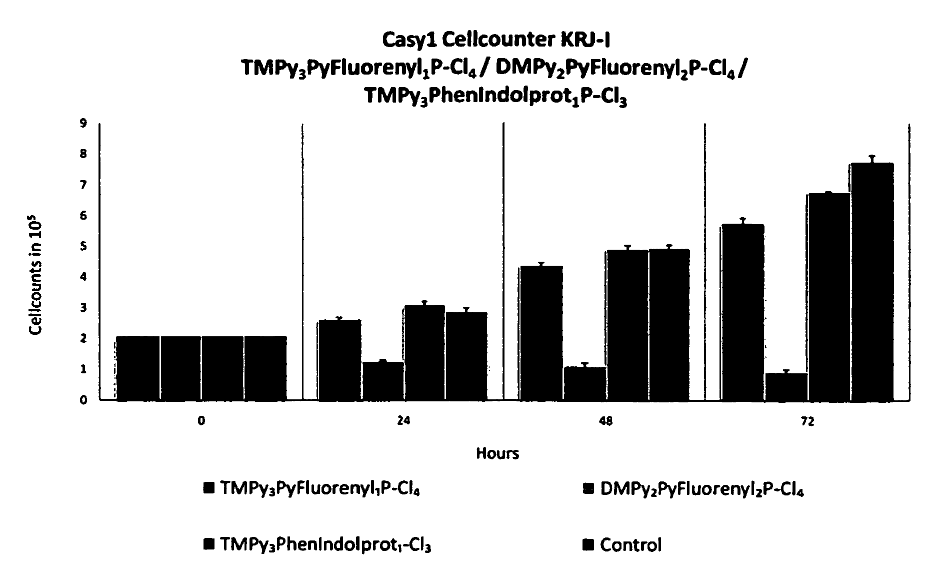

Casy1 cellcounts of KRJ-I cells at a drug application of 10 µg/ml porphyrin.

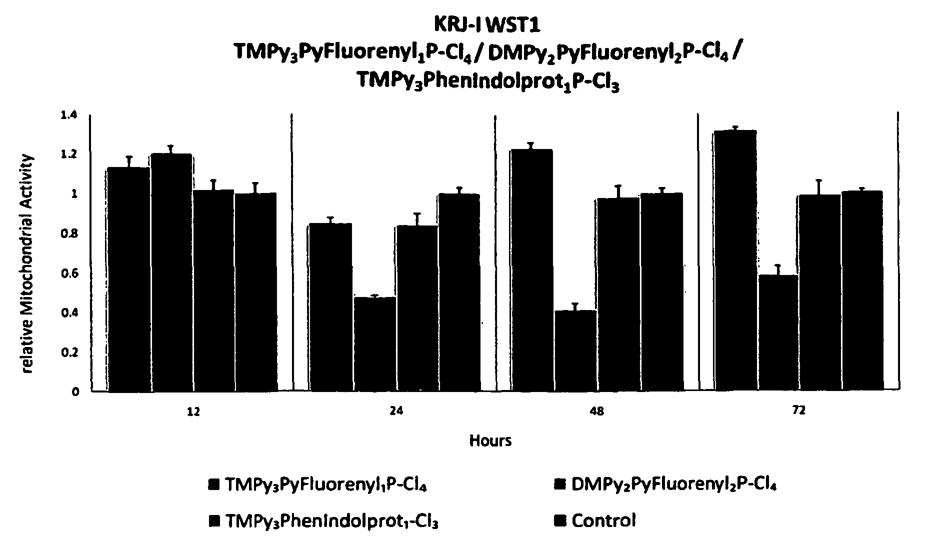

Mitochondrial activity of KRJ-I cells at a drug application of 10 µg/ml porphyrin.

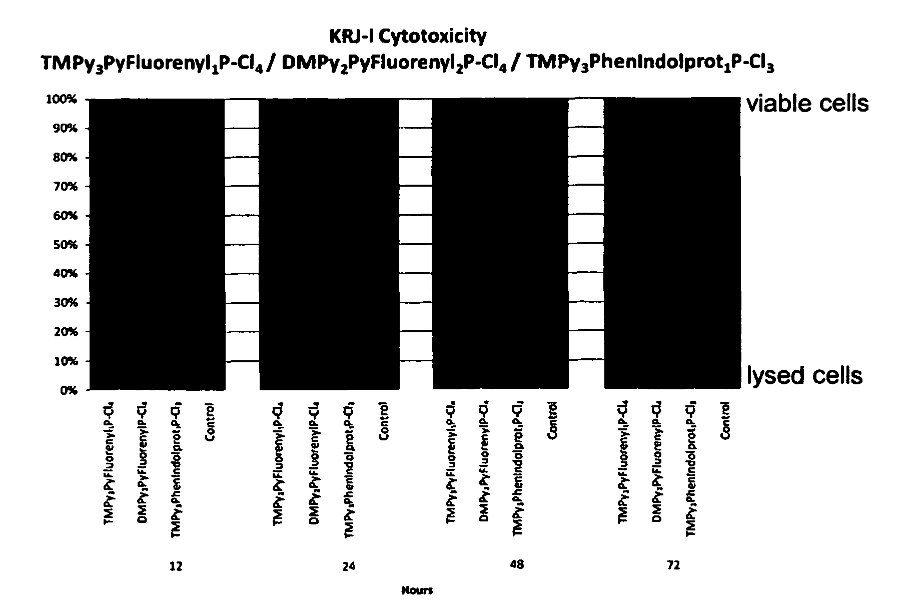

Cytotoxicity on KRJ-I cells at a drug application of 10 µg/ml porphyrin.

The cytotoxicity, WST-1 mitochondrial activity and the Casy1 cell count tests showed that the compound TMPy₃PyFluorenyl₁P-Cl₄ induced a significantly lower cytotoxicity than DMPy₂PyFluorenyl₂P-Ck₄ on the KRJ-I cancer cell line.

As a consequence it is supposed that porphyrins with a trans symmetry of their fluorenyl modified pyridyl groups increase cytotoxicity tremendously in comparison to porphyrins with only one fluorenyl modified pyridyl substituent.

The cytotoxicity for KRJ-I, induced by the application of porphyrin compounds, decreases in the following order: DMPy₂PyFluorenyl₂P-Cl₄ > TMPy₃Phenlndolprot₁P-Cl₃ > TMPy₃PyFluorenyl₁P-Cl₄.

Cytotoxicity, Casy1 Cell counts and WST-1 mitochondrial activity test of MTC-SK tumor cells upon incubation with porphyrins

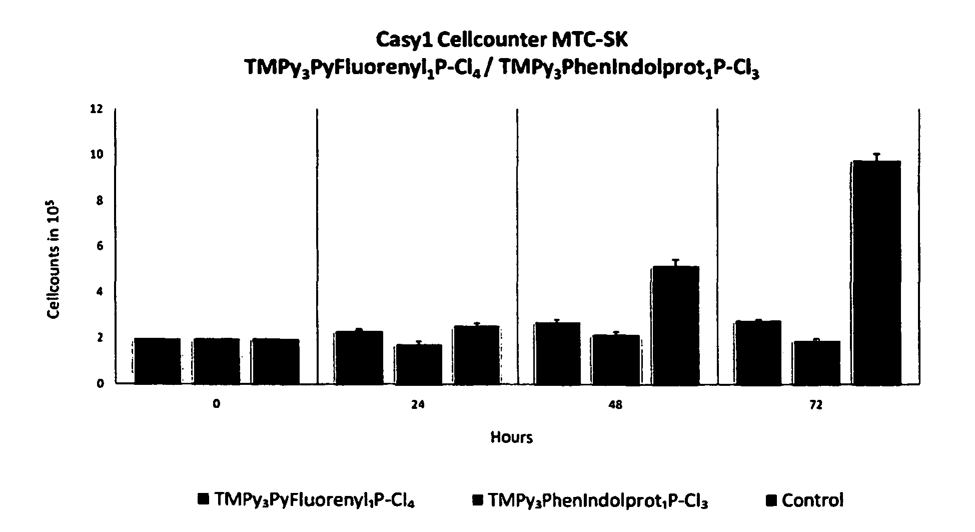

Mitochondrial activity of MTC-SK cells at a drug application of 10 µg/ml.

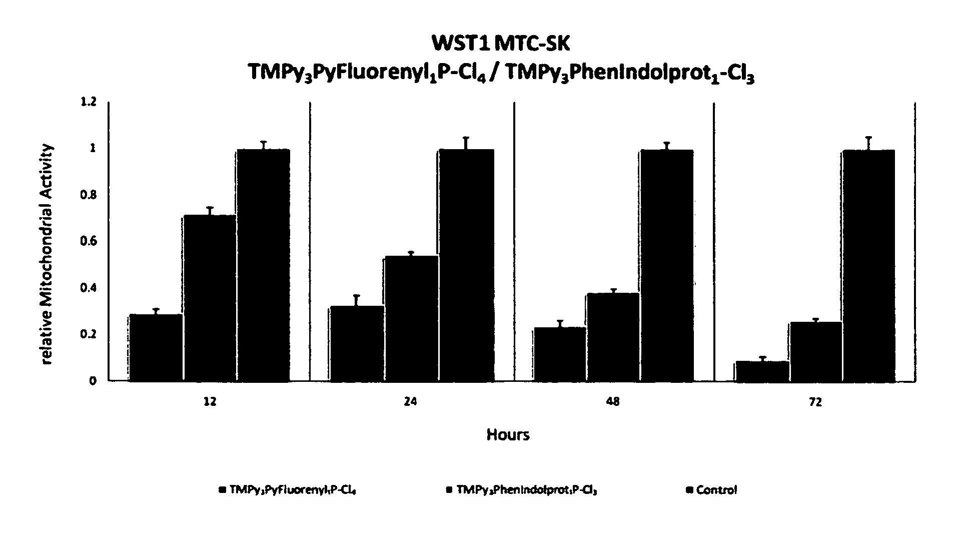

Mitochondrial activity of MTC-SK cells at a drug application of 10 µg/ml.

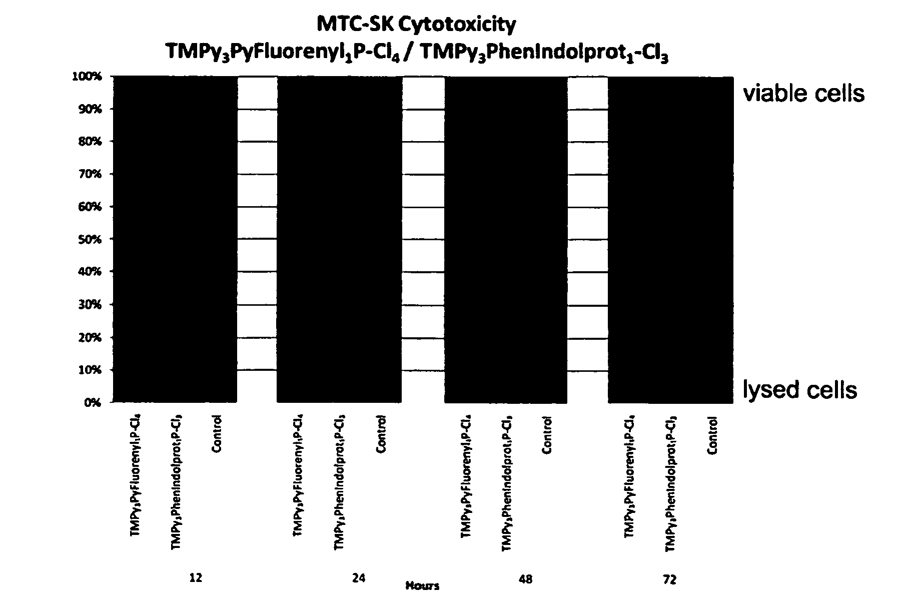

Cytotoxicity on MTC-SK cells at a drug application of 10 µg/ml porphyrin.

All the tests shown above depict, that both porphyrin compounds inhibit cell growth if incubated with MTC-SK cancer cells. TMPy₃Phenlndolprof₁P-C1₃ shows significantly stronger cell growth inhibition than TMPy₃PyFluorenyl₁P-C<₄. Therefore it is clear that the compound TMPy₃Phenlndolprot₁P-Cb is a potent drug to destroy MTC-SK carcinoma cells.

### Examples:

### Example 1

### 5,10,15-tris(4-pyridyl)-20-(4-bromophenyl)-21H,23H-porphyrin (TPy₃PhenBr₁P)

To a refluxing mixture of 50 ml propionic acid and 10 ml propionic anhydride 1.5 mmol 4-bromobenzaldehyde, 4.5 mmol 4-pyridinecarboxaldehyde and 6.2 mmol of freshly distilled pyrrole are added. This mixture is refluxed for 120 min. Then 2.0 mmol 2,3-dichloro-5,6-dicyano-1,4-benzoquinone (DDQ) is added and the mixture is stirred over night at room temperature. Then the solvent is evaporated to dryness and the black-purple product is dissolved in CHCl₃. This mixture is divided into several parts and neutralized with saturated NaHCO₃ water solution. The neutralized solution is dried over MgSO₄, filtered and evaporated to dryness. Due to the fact that during this asymmetric synthesis several porphyrin products are obtained, intensive column chromatography is needed to separate the different porphyrin species. Silica gel is used as stationary phase and the products are eluted with a mixture of CHCl₃ MeOH 19:1 (v/v) and CHCl₃ : MeOH 49:1 (v/v). The desired crude product is the fifth fraction on the column. Column chromatography is repeated several times with different mixtures of CHCl₃ MeOH v/v to finally obtain pure compounds. The desired compound is chosen, after mass spectrometer and NMR analysis of each obtained compound. *Yield:* 4 %
**¹H-NMR** (500 MHz, CDCl₃, 303.57 K, δ ):
9.06 (d, J = 4.85, 6H, pyridyl-3,5-H), 8.90 (d, J = 4.65, 2H, □-pyrrole-H), 8.86 (s, 4H, β-pyrrole-H), 8.84 (d, J = 4.65, 2H, β-pyrrole-H), 8.17 (d, J = 4.85, 6H, pyridyl-2,6-H), 8.07 (d, J = 7.99, 2H, phenyl-3,5-H), 7.97 (d, J = 7.99, 2H, phenyl-2,6-H), -2.88 (s, 2H, pyrrole-NH)
**¹³C-NMR** (125 MHz, CDCl₃, 303.57 K, δ ):
150.2 (s, 3C, quart. ipso pyridyl-1-C), 150.2 (s, 1C, quart. ipso phenyl-1-C), 148.5 (d, 6C, pyridyl-3,5-CH), 131.9 (d, 2C, β-pyrrole-CH), 131.3 (d, 2C, □ -pyrrole-CH), 131.2 (d, 4C, □-pyrrole-CH), 140.6 (s, 8C; β-pyrrole-C), 135.9 (d, 2C, phenyl-2,6-CH), 130.3 (d, 2C, phenyl-3,5-CH), 129.5 (d, 6C, pyridyl-2,6-CH), 123.1 (s, 1C, quart. meso phenyl-1-C), 120.2 (s, 1C, quart. para phenyl-4-C), 117.7 (s, 2C, quart. meso pyridyl-C), 117.5 (s, 1C, quart. meso pyridyl-C)
**ESI-MS** (Chloroform/Methanol 1:1, positive ion mode): m/z = 698.33 ([M+H]⁺) C₄₁H₂₆BrN₇ theoretical mass: 695.14, 697.14, 696.14

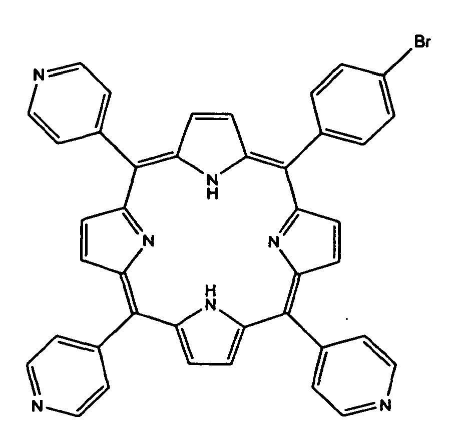

### Example 2:

### 5,10,15-tris(4-pyridyl)-20-(1-phenyl-4-(3-(N-phenylsulfonyl-)indolyl)-21H,23H-porphyrin (TPy₃Phenlndolprot₁P)

43.0 µmol TPy₃PhenBr₁P and 86.1 µmol 1-(phenylsulfonyl)-3-indoleboronic acid are dissolved in 40 ml toluene and 10 ml methanol. Then the mixture is degassed and saturated with argon. After that 1.2 µmol tetrakis(triphenylphosphine)palladium(0) and 1 ml 2 M Na₂CO₃ solution are added under argon atmosphere. Then the reaction mixture is protected from light, heavily stirred and heated up to 80 °C for 90 h. After that, the reaction mixture is filtered, extracted with deionized water, dried over Na₂SO₄ and evaporated to dryness. For the purification of the crude product, intensive column chromatography is needed. Silica gel is used as stationary phase and the product is eluted using a mixture of CHCl₃ : MeOH 69:1 (v/v). The second band is the desired product, which is evaporated to dryness after the separation process. *Yield:* 79 %. The third band eluting form the column was identified as 5,10,15-tris(4-pyridyl)-20-(1-phenyl-4-(3-indolyl-1H)-21H,23H-porphyrin (14) using mass spectrometer analysis. *Yield:* 12 %
**¹H-NMR** (500 MHz, CDCl₃, 303.57 K, δ ):
9.07 (d, J = 4.42, 6H, pyridyl-3,5-H), 9.02 (d, J = 4.51, 2H, □-pyrrole-H), 8.86 (s, 4H, β-pyrrole-H), 8.84 (d, J = 4.51, 2H, □-pyrrole-H), 8.31 (d, J =7.24, 2H, phenyl-3,5-H), 8.21 (d, J = 4.42, 6H, pyridyl-2,6-H), 8.19 (d, J = 8.65, 1H, indolyl-7-H), 8.11 (d, J = 7.75, 1H, indolyl-4-H), 8.05 (d, J =7.24, 2H, phenyl-2,6-H), 8.05 (d, J = 4.26, 2H, phenylsulfonyl-2,6-H), 8.04 (s, 1H, indolyl-2-H), 7.62 (q, J = 7.62, 1H, phenylsulfonyl-4-H), 7.54 (t, J = 7.62, 2H, phenylsulfonyl-3,5-H), 7.49 (m, 1H, indolyl-6-H), 7.44 (m, 1H, indolyl-5-H), -2.84 (s, 2H, pyrrole-NH)
**¹³C-NMR** (125 MHz, CDCl₃, 303.57 K, δ ):
150.8 (s, 3C, quart. ipso pyridyl-1-C), 150.8 (s, 1C, quart. ipso phenyl-1C), 148.0 (d, 6C, pyridyl-3,5-CH), 140.8 (s, 1C, quart. indolyl-3-C), 138.5 (s, 1C, quart. phenylsulfonyl-1-C), 136.0 (s, 8C, quart. β-pyrrole), 135.3 (d, 2C, phenyl-2,6-CH), 134.2 (d, 1C, phenylsulfonyl-4-CH), 133.2 (s, 1C, quart. phenyl-4-C), 132.4 (d, 2C, β-pyrrole-CH), 131.0 (d, 4C, β-pyrrole-CH), 130.9 (d, 2C, β -pyrrole-CH), 129.7 (d, 6C, pyridyl-2,6-CH), 129,6 (d, 2C, phenylsulfonyl-3,5-CH), 129.5 (s, 1C, quart. indol-7a-C), 127.1 (d, 2C, phenylsulfonyl-2,6-CH), 126.5 (d, 2C, phenyl-3,5-CH), 125.5 (d, 1C, indolyl-6-CH), 124.1 (d, 1C, indolyl-5-CH), 123,7 (s, 1C, quart. indolyl-3a-C), 123.7 (d, 1C, indolyl-3-CH), 121.5 (s, 1C, quart. meso phenyl-C), 120.8 (d, 1C, indolyl-4-CH), 117.5 (s, 2C, quart. meso pyridyl-C), 117.2 (s, 1C, quart. meso pyridyl-C), 114.2 (d, 1C, indolyl-7-CH)

### ESI-MS (Chloroform/Methanol 1:1, positive ion mode): m/z = 873.34 ([M+H]⁺). C₅₅H₃₆N₈O₂S theoretical mass = 872.27

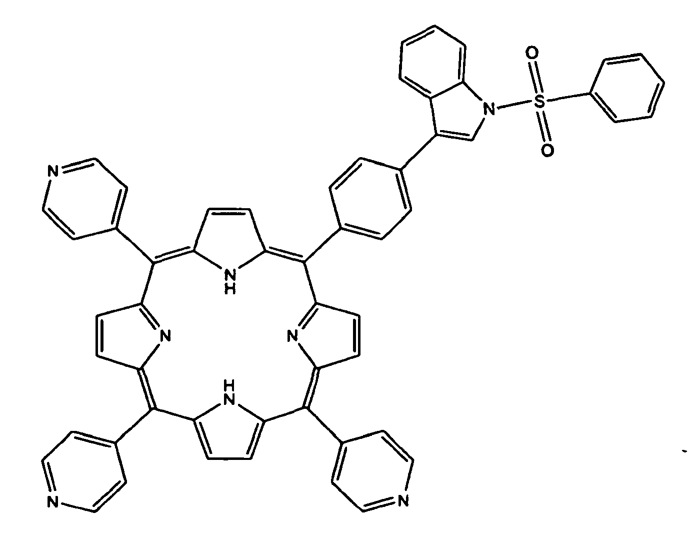

### Example 3:

### 5,10,15-tris(4-pyridyl)-20-(1-phenyl-4-(3-indolyl)-21H,23H-porphyrin (TPy₃Phenlndoldeprot₁P)

43.0 µmol TPy₃PhenBr₁P and 86.1 µmol 1-(phenylsulfonyl)-3-indoleboronic acid are dissolved in 40 ml toluene and 10 ml methanol. Then the mixture is degassed and saturated with argon. After that 1.2 µmol tetrakis(triphenylphosphine)palladium(0) and 1 ml 2 M Na₂CO₃ solution are added under argon atmosphere. Then the reaction mixture is protected from light, heavily stirred and heated up to 80 °C for 90 h. After that, the reaction mixture is filtered, extracted with deionized water, dried over Na₂SO₄ and evaporated to dryness. For the purification of the crude product intensive, column chromatography is needed. Silica gel is used as stationary phase and the product is eluted using a mixture of CHCl₃ : MeOH 69:1 (v/v). The third band is the desired product. *Yield: 12* %
**¹H-NMR** (500 MHz, CDCl₃, 303.57 K, δ ):
9.09 (m, 2H, β-pyrrole-H), 9.08 (m, 6H, pyridyl-3,5-H), 8.87 (s, 4H, β-pyrrole-H), 8.84 (m, 2H, β-pyrrole-H), 8.55 (s, 1H, indolyl-1-NH), 8.28 (d, J = 7.61, 2H, phenyl-3,5-H), 8.24 (d, J = 7.28, 1H, indolyl-7-H), 8.18 (m, 6H, pyridyl-2,6-H), 8.10 (d, J = 7.61, 2H phenyl-2,6-H), 7.68 (bs, 1H, indolyl-2-H), 7.55 (m, 1H, indolyl-4-H), 7.35 (m, 2H, indolyl-5,6-H), -2.82 (s, 2H, pyrrole-NH)
**¹³C-NMR** (125 MHz, CDCl₃, 303.57 K, δ ):
150.3 (s, 3C, quart. ipso-pyridyl-1-C), 150.2 (s, 1C, quart. ipso-phenyl-1-C), 148.5 (d, 6C, pyridyl-3,5-CH), 139.1 (s, 8C, quart. α-pyrrole-C), 137.1 (s, 1C, quart. indolyl-C), 135.7 (s, 1C, quart. indolyl-C), 135.3 (d, 2C, phenyl-3,5-CH), 132.6 (d, 2C, □-pyrrole-CH), 132.5 (d, 2C, β-pyrrole-CH), 131.0 (d, 2C, □ -pyrrole-CH), 129.6 (d, 6C, pyridyl-2,6-CH), 126.0 (s, 1C, quart. indolyl-C), 125.9 (d, 2C, phenyl-2,6-CH), 122.9 (d, 1C, indolyl-5-CH), 122.5 (d, 1C, indolyl-2-CH), 122.2 (s, 1C, quart. phenyl-4-C), 120.8 (d, 1C, indolyl-6-CH), 120.1 (d, 1C, indolyl-7-CH), 118.0 (s, 1C, meso phenyl-C), 117.6 (s, 2C, quart. meso pyridyl-C), 117.1 (s, 1C, quart. meso pyridyl-C), 111.8 (d, 1C, indolyl-4-CH)
**ESI-MS** (Chloroform/Methanol 1:1, positive ion mode): m/z = 733.67 ([M+H]⁺). C₄₉H₃₂N₈ theoretical mass = 732.83

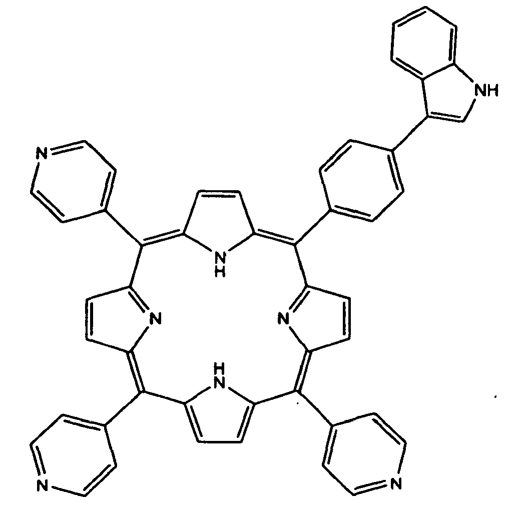

### Example 4

### 5,10,15-tris(N-methylpyridinium-4-yl)-20-(1-phenyl-4-(3-(N-phenylsulfonyl)-indolyl)-21H,23H-porphyrin chloride (TMPy₃Phenlndolprot₁P-Ct₃)

To a mixture of 10 ml CH₃NO₂ and 10 ml CHCl₃ (1:1 v/v) 9.2 µmol TPy₃PhenlndolPhen₁P and 0.676 mmol methyl p-toluene-sulfonate are added. This reaction mixture is refluxed for 90 h under argon atmosphere. The progress of the N-methylation reaction can be monitored using silica gel TLC and an eluent containing ACN : H₂O (sat. KNO₃) : H₂O 8:1:1 (v/v). Then the reaction mixture is evaporated to dryness, dissolved in deionized water and the impurities are extracted with CHCl₃. The remaining water fraction is filtered and then evaporated to dryness which gives the tosylate salt of TMPy₃PhenlndolPhen₁P³⁺.
TMPy₃PhenlndolPhen₁P-tos₃ is dissolved in 35 ml H₂O and 5 ml MeOH. Then 2 g of Dowex 1x8 Cl⁻ ion exchange resin are added. After 10 h of slow stirring, the ion exchange resin is filtered off and the remaining reaction mixture is evaporated to dryness, which gives the Cl⁻ salt of TMPy₃PhenlndolPhen₁P³⁺. *Yield:* 90 %

**¹H-NMR** (500 MHz, CDCl₃, 303.57 K, δ ):
9.40 (d, J = 5.13, 6H, pyridyl-3,5-H), 9.11 (m, 8H, β-pyrrole-H), 8.96 (d, J = 5.13, 6H, pyridyl-2,6-H), 8.93 (d, J = 5.70, 2H, phenylsulfonyl-2,6-H y), 8.59 (t, J = 7.36, 1H, phenylsulfonyl-4-H y), 8.29 (d, J = 7.23, 2H, phenyl-2,6-H), 8.13 (s, 1H, indolyl-2-H), 8.10 (m, 2H, phenylsulfonyl-3,5-H y), 8.08 (m, 1H, indolyl-7-H), 8.06 (m, 2H, phenyl-3,5-H), 8.06 (m, 2H, phenylsulfonyl-2,6-H x), 8.03 (d, J = 8.02, 1H, indolyl-4-H), 7.66 (t, J = 7.39, 1H, phenylsulfonyl-4-H x), 7.58 (t, J = 7.39, 2H; phenylsulfonyl-3,5-H), 7.42 (m, 1H, indolyl-6-H), 7.36 (m, 1H, indolyl-5-H), 4.85 (s, 9H, N⁺-CH₃), -2,83 (s, 2H, pyrrole-NH)

**¹³C-NMR** (125 MHz, CDCl₃, 303.57 K, δ ):
159.7 (s, 2C, quart. ipsb pyridyl-1-C), 159.7 (s, 1C, quart. ipso phenyl-1-C), 152.8 (s, 8C, quart. α-pyrrole-C), 146.7 (d, 1C, phenylsulfonyl-4-CH y), 145.9 (d, 2C, phenylsulfonyl-2,6-CH y), 145.5 (d, 6C, pyridyl-3,5-CH), 141.3 (s, 1C, quart. indolyl-3-C), 139.1 (s, 1C, quart. ipso phenylsulfonyl-1-C y), 137.1 (s, 1C, quart. ipso phenylsulfonyl-1-C x), 136.4 (d, 2C, phenyl-2,6-CH), 135.5 (d, 1C, phenylsulfonyl-4-CH x), 134.9 (s, 1C, quart. phenyl-4-C), 134.1 (d, 6C, pyridyl-2,6-CH), 132.5 (d, 8C, β-pyrrole-CH), 132.1 (s, 1C, quart. indolyl-7a-C), 130.7 (d, 2C, phenylsulfonyl-3,5-CH x), 129.2 (d, 1C, indolyl-7-CH), 128.1 (d, 2C, phenyl-2,6-CH), 128.1 (d, 2C, phenylsulfonyl-2,6-CH x), 126.3 (d, 1C, indolyl-4-CH), 125.1 (d, 1C, indolyl-2-CH), 125.1 (d, 1C, indolyl-6-CH), 124.8 (s, 1C, quart. indolyl-3a-C), 121.6 (d, 1C, indolyl-5-CH), 120.8 (s, 1C, quart. meso phenyl-1-C), 117.3 (s, 2C, meso pyridyl-1-C), 116.4 (s, 1C, meso pyridyl-1-C), 115.1 (d, 2C, phenylsulfonyl-3,5-CH y), 49.1 (q, 3C, N⁺-CH₃)

**ESI-MS** (Methanol 1:1, positive ion mode): m/z = 305.87 ([M]³⁺), 458.20 ([M]²⁺), 544.07 ([M+tos]²⁺)_{.}
C₅₈H₄₅N₈O₂ S³⁺ theoretical mass = 917.34

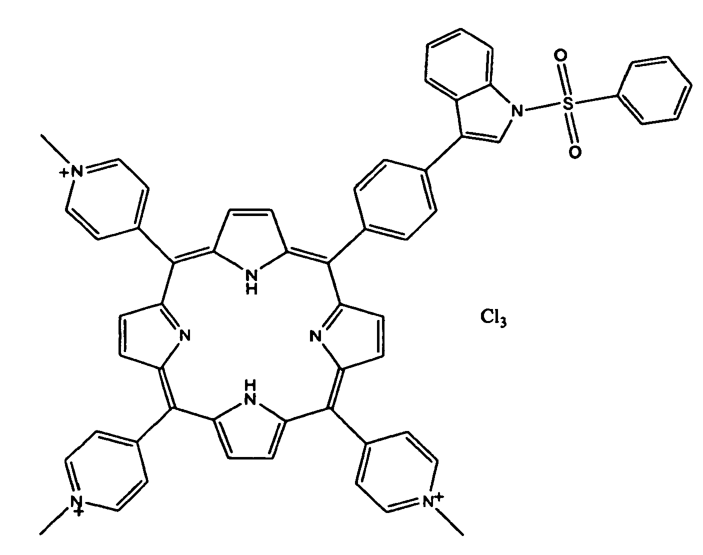

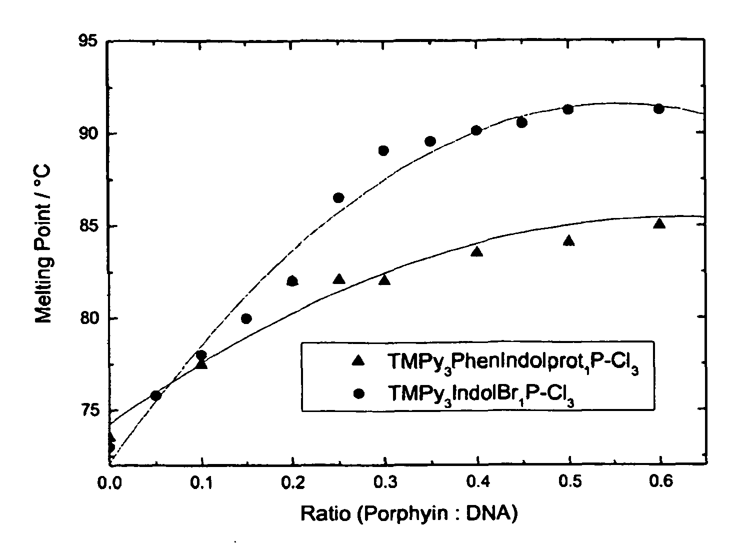

Comparison of the melting points of CT-DNA complexed with different indolyl modified porphyrins. (0.06 mM CT-DNA in 5 mM (CH₃)₂AsO₂Na, 20 mM NaCl, pH 7.0 buffer)

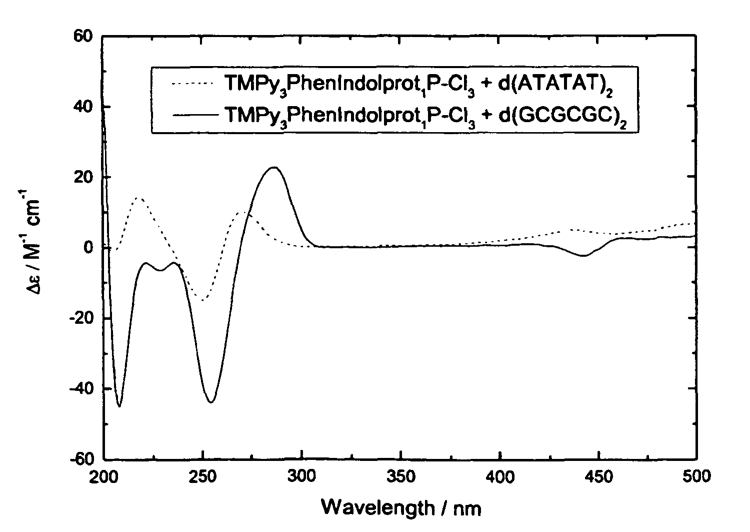

CD-spectra of the complex of TMPy₃Phenlndolprot₁P-Cl₄ with d(GCGCGC)₂ (B-DNA) / d(ATATAT)₂ (A-DNA). (0.2 mM DNA, 0.2 mM porphyrin in 5 mM (CH₃)₂AsO₂Na, 20 mM NaCl, pH 7.0 buffer (d(GCGCGC)₂ at 20 °C; d(ATATAT)₂ at 5 °C)

The calf-thymus (CT) DNA melting temperature figure shows that the compound TMPy₃Phenlndolprot₁P-Cl₄ interacts with CT-DNA and stabilizes its double strand. Next to that the additional circular dichroism spectra show that TMPy₃Phenlndolprof₁P-Cl₄ intercalates B-DNA while it shows outside binding with A-DNA. Therefore the binding mode of this porphyrin drug to A- and B-DNA next to its duplex DNA stabilization is proven.

### Example 5:

### 5,10,15-tris(N-methylpyridinium-4-yl)-20-(1-phenyl-4-(3-indolyl)-21H,23H-porphyrin chloride (TMPy₃Phenlndoldeprot₁P-Cl₃)

To a mixture of 5 ml CH₃NO₂ and 5 ml CHCl₃ (1:1 v/v) 6.3 µmol TPy₃Phenlndoldeprot₁P and 0.476 mmol methyl p-toluene-sulfonate are added. This reaction mixture is refluxed for 118 h under argon atmosphere. The progress of the N-methylation reaction can be monitored using silica gel TLC and an eluent containing ACN : H₂O(sat. KNO₃) : H₂O 8:1:1 (v/v). Then the reaction mixture is evaporated to dryness, dissolved in deionized water and the impurities are extracted with CHCl₃. The remaining water fraction is filtered and then evaporated to dryness, which gives the tosylate salt of TMPy₃Phenlndoldeprot₁P³⁺. TMPy₃Phenindoldeprot₁P-tos₃ is dissolved in 35 ml H₂O and 5 ml MeOH. Then 2 g of Dowex 1x8 Cl⁻ ion exchange resin are added. After 12 h of slow stirring, the ion exchange resin is filtered off and the remaining reaction mixture is evaporated to dryness, which gives the Cl⁻ salt of TMPy₃Phenlndoldeprot₁P³⁺. Yield: 87 %

**¹H-NMR** (500 MHz, CDCl₃, 303.57 K, δ ):
9.38 (d, J = 5.95, 4H, pyridyl-3,5-H), 9.35 (d, J = 5.33, 2H, pyridyl-3,5-H), 9.11 (s, 4H, β-pyrrole-H), 9.00 (s, 4H, β-pyrrole-H), 8.94 (m, 4H, pyridyl-2,6-H), 8.90 (m, 2H, pyridyl-2,6-H), 8.52 (s, 1H, indolyl-NH), 8.17 (d, J = 5.90, 2H, phenyl-3,5-H), 8.08 (s, 1H, indolyl-2-H), 8.08 (m, 2H, phenyl-2,6-H), 7.77(m, 1H, indolyl-7-H), 7.64 (d, J = 7.87, 6H, tosylate-3,5-H) 7.54 (d, J = 8.27, 1H, indolyl-4-H), 7.23 (m, 1H, indolyl-6-H), 7.17 (d, J = 7.87, 6H, tosylate-2,6-H) 7.15 (m, 1H, indolyl-5-H), 4.79 (s, 9H, N⁺-CH₃), 2.29 (s, 9H, tosylate-CH₃), -2.20 (s, 2H, pyrrole-NH)

**¹³C-NMR** (125 MHz, CDCl₃, 303.57 K, δ [ppm]):
159.1 (s, 3C, quart. ipso pyridyl-1-C), 159.1 (s, 1C, ipso-Phenyl-1-C), 145.7 (d, 4C, pyridyl-3,5-CH), 145.5 (d, 2C, pyridyl-3,5-CH), 143.5 (s, 3C, quart. tosylate-1-C), 141.6 (s, 3C, quart. tosylate-4-C), 140.0 (s, 8C, α-pyrrole-C), 139.0 (s, 1C, quart. indolyl-7a-C), 138.5 (s, 1C, quart. indolyl-3a-C), 137.2 (d, 2C, phenyl-3,5-CH), 134.9 (d, 4C, pyridyl-2,6-CH), 134.2 (d, 2C, pyridyl-2,6-CH), 132.7 (d, 4C, β -pyrrole-CH), 131.4 (d, 4C, β-pyrrole-CH), 129.8 (d, 6C, tosylate-2,6-CH), 126.9 (d, 6C, tosylate-3,5-CH), 126.8 (d, 2C, phenyl-2,6-CH), 125.0 (d, 1C, indolyl-7-CH), 123.1 (d, 1C, indolyl-6-CH), 122.8 (s, 1C, quart. phenyl-4-C), 121.2 (d, 1C, indolyl-5-CH), 120.3 (d, 1C, indolyl-2-CH), 116.7 (s, 1C, quart. indolyl-3-C), 117.0 (s, 1C, quart. meso phenyl-C), 115.7 (s, 3C, quart. meso pyridyl-C), 113.1 (d, 1C, indolyl-4-CH), 49.0 (q, 3C, N⁺-CH₃), 21.2 (q, 3C, tosylate-CH₃)

**ESI-MS** (Methanol 1:1, positive ion mode): m/z = 259.33 ([M]³⁺), 388.93 ([M]²⁺) C₅₂H₄₁N₈³⁺ theoretical mass = 777.93

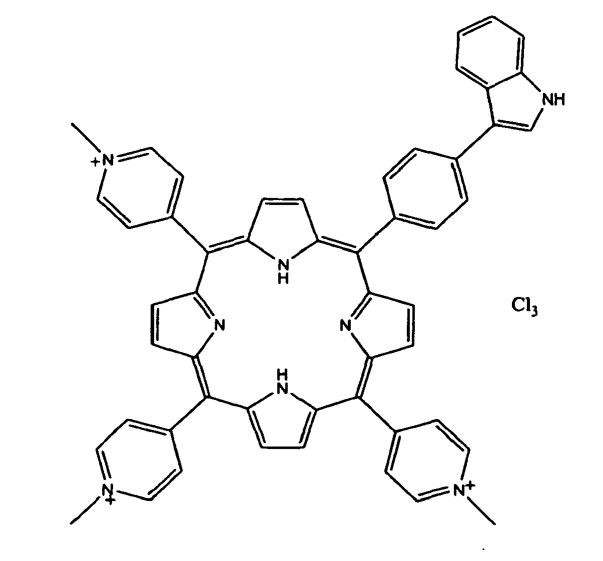

### Mono meso-N-methylpyridinium-3-yl substituted porphyrins modified with 9,9-dimethylfluorene

### Example 6

### Synthesis of 5-(5-bromo-3-pyridyl)-10,15,20-tris(4-pyridyl)-21H,23H-porphyrin ("TPy₃PyBr₁P")

To a refluxing mixture of 50 ml propionic acid and 10 ml propionic anhydride 1.5 mmol 5-bromo-3-pyridinecarboxaldehyde, 4.5 mmol 4-pyridinecarboxaldehyde and 6.2 mmol of freshly distilled pyrrole are added. This mixture is refluxed for 150 min. Then the reaction mixture is cooled to room temperature and 2 mmol of 2,3-dichloro-5,6-dicyano-1,4-benzoquinone (DDQ) are added. The mixture is stirred for 1 h, then the solvent is evaporated to dryness and the black-purple product is dissolved in CHCl₃. This mixture is divided into several parts and neutralized with a saturated NaHCO₃ water solution. The neutralized solution is dried over MgSO₄, filtered and evaporated to dryness. Due to the fact that during this statistical synthesis several porphyrin products are obtained, intensive column chromatography is needed to separate the different porphyrin species. Silica gel is used as stationary phase and the products are eluted with a mixture of CHCl₃ : MeOH 19:1 (v/v). The column chromatography is repeated several times with different mixtures of CHCl₃ MeOH v/v to finally obtain pure compounds. The desired compound is chosen, after mass spectrometer and NMR analysis of each obtained compound. *Yie*/*d*. 6 %

**¹H-NMR** (500 MHz, CDC13, 303.57 K, δ ):
9.37 (d, J = 1.88, 1H, pyridyl-4-a-H), 9.15 (d, J = 1.88, 1H, pyridyl-6-a-H), 9.07 (d, J = 5.35, 6H, pyridyl-3,5-b-H), 8.88 (m, 8H, β-pyrrole-H), 8.69 (m, 1H, pyridyl-2-α-H), 8.17 (d, J = 5.35, 6H, pyridyl-2,6-b-H), -2.93 (s, 2H, pyrrole-NH)

**¹³C-NMR** (125 MHz, CDCl₃, 303.57 K, δ ):
151.8 (d, 1C, pyridyl-4-α-CH), 150.7 (d, 1C, pyridyl-6-a-CH), 149.8 (s, 3C, ipso pyridyl-1-b-C), 148.6 (d, 6C, pyridyl-3,5-b-CH), 146.8 (s, 8C, quart. α -pyrrole-C), 143.3 (d, 1C, pyridyl-2-α-CH), 139.1 (s, 1C, quart. ipso pyridyl-1-a-C), 131.5 (d, 8C, β-pyrrole-CH), 129.5 (d, 6C, pyridyl-2,6-b-CH), 120.0 (s, 1C, quart. meso pyridyl-a-C), 118.1 (s, 3C, quart. meso pyridyl-b-C), 115.5 (s, 1C, quart. pyridyl-5-a-C)

**ESI-MS** (Chloroform/Methanol 1:1, positive ion mode): m/z = 697.53 ([M+H]⁺), 699.53 ([M+H]⁺), 698.53 ([M+H]⁺) C₄₀H₂₅BrN₈ theoretical mass = 696.14, 698.14, 697.14

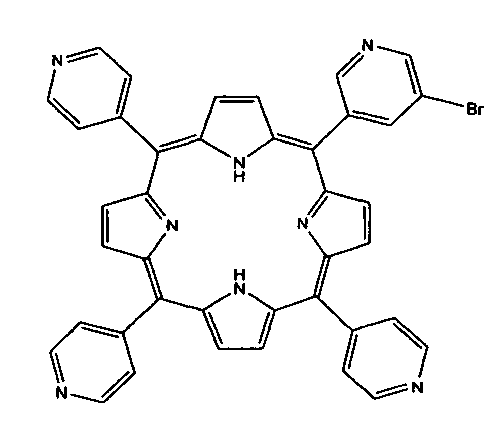

### Example 7

### 5-(5-(2-(9,9-dimethyl)fluorenyl)-3-pyridyl)-10,15,20-tris(4-pyridyl)-21 H,23H-porphyrin ("TPy3PyFIuorenyl₁P")

30.0 µmol TPy3PyBr₁P and 60.0 µmol 9,9-dimethylfluorenyl-2-boronic acid dimethylester are dissolved in 40 ml toluene and 10 ml methanol. Then the mixture is degassed and saturated with argon. After that 0.9 µmol tetrakis(triphenylphosphine)-palladium(0) and 0.8 ml 2 M Na₂CO₃ solution are added under argon atmosphere. Then the reaction mixture is protected from light, heavily stirred and heated up to 80 °C for 47 h. After that, the reaction mixture is filtered, extracted with deionized water, dried over Na₂SO₄ and evaporated to dryness. For the purification of the crude product, intensive column chromatography is needed. Silica gel is used as stationary phase and the product is eluted using a mixture of CHCl₃ : MeOH 29:1 (v/v). The second band is the desired product, which is evaporated to dryness after the separation process. *Yield:* 82 %

**¹H-NMR** (500 MHz, CDCl₃, 303.57 K, δ ):
9.43 (d, J = 2.01, 1H, pyridyl-4-a-H), 9.39 (d, J = 2.01, 1H, pyridyl-6-a-H), 9.07 (d, J = 5.45, 6H, pyridyl-3,5-b-H), 8.99 (d, J = 4.92, 2H, β-pyrrole-H), 8.92 (m, 2H, β-pyrrole-H), 8.88 (s, 4H, □-pyrrole-H), 8.79 (m, 1H, Pyridyl-2-a-H), 8.18 (d, J = 5.45, 6H, Pyridyl-2,6-b-H), 7.92 (s, 1H, fluorenyl-H), 7.88 (m, 1H, fluorenyl-H), 7.87 (m, 1H, fluorenyl-H), 7.78 (m, 1H, fluorenyl-H), 7.44 (m, 1H, fluorenyl-H), 7.38 (m, 1H, fluorenyl-H), 7.36 (m, 1H, fluorenyl-H), 1.55 (s, 6H, fluorenyl-CH₃), -2.84 (s, 2H, pyrrole-NH)

**¹³C-NMR** (125 MHz, CDCl₃, 303.57 K, δ ):
154.6 (s, 2C, quart. fluorenyl-8a, 9a-C), 152.1 (d, 1C, pyridyl-4-a-CH), 150.0 (s, 3C, quart. ipso pyridyl-1-b-C), 148.6 (d, 6C, pyridyl-3,5-b-CH), 147.0 (s, 8C, □ -pyrrole-C), 148.0 (d, 1C, pyridyl-6-a-CH), 139.3 (d, 1C, pyridyl-2-a-CH), 137.6 (s, 1C, quart ipso-pyridyl-1-a-C), 136.3 (s, 1C, quart. pyridyl-5-a-C), 135.6 (s, 2C, quart. fluorenyl-4a,4b-C), 131.6 (d, 8C, β-pyrrole-CH), 129.5 (d, 6C, pyridyl-2,6-b-CH), 128.0 (d, 1C, Fluorenyl-CH), 127.4 (d, 1C, fluorenyl-CH), 126.8 (d, 1C, fluorenyl-CH), 122.9 (d, 1C, fluorenyl-CH), 121.8 (d, 1C, fluorenyl-CH), 121.0 (d, 1C, fluorenyl-CH), 120.5 (d, 1C, fluorenyl-CH), 118.0 (s, 3C, quart. meso pyridyl-1-b-C), 118.0 (s, 1C, quart. meso pyridyl-1-a-C), 117.2 (s, 1C, quart. ipso-fluorenyl-2-C), 47.3 (s, 1C, quart. fluorenyl-9-C), 27.4 (q, 2C, fluorenyl-CH₃)

**ESI-MS** (Chloroform/Methanol 1:1, positive ion mode): m/z = 811.61 ([M+H]⁺). C₅₅H₃₈N₈ theoretical mass = 810.32

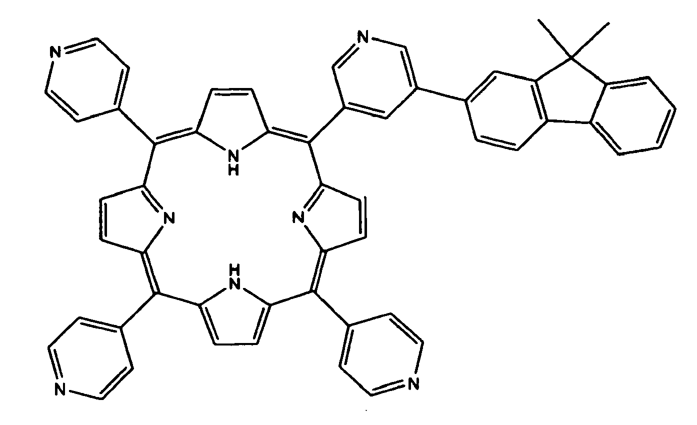

### Example 8

### 5-(5-(2-(9,9-dimethyl)fluorenyl)-N-methylpyridinium-3-yl)-10,15,20-tris(N-methylpyridinium-4-yl)-21H,23H-porphyrin tosylate ("TMPy₃PyFluorenyl₁P-tos₄")

To a mixture of 10 ml CH₃NO₂ and 10 ml CHCl₃ (1:1 v/v) 8.5 µmol TPy₃PyFluorenyl₁P and 0.85 mmol methyl p-toluene-sulfonate are added. This reaction mixture is refluxed for 87 h under argon atmosphere. The progress of the N-methylation reaction can be monitored using silica gel TLC and an eluent containing ACN : H₂O (sat. KNO₃) : H₂O 8:1:1 (v/v).
Then the reaction mixture is evaporated to dryness and dissolved in deionized water and the impurities are extracted with CHCl₃. The remaining water fraction is filtered and then evaporated to dryness, which gives the tosylate salt of TMPy3PyFluorenyl₁P⁴⁺. *Yield:* 97 %

**¹H-NMR** (500 MHz, CD₃OD, 303.57 K, δ ):
9.92 (s, 1H, pyridyl-4-a-H), 9.89 (s, 1H, pyridyl-6-a-H), 9.75 (s, 1H, pyridyl-2-aH), 9.42 (bs, 6H, pyridyl-3,5-b-H), 9.33 (bs, 2H, β-pyrrole-H), 9.21 (s, 4H, β -pyrrole-H), 9.17 (bs, 2H, β-pyrrole-H), 8.99 (bs, 6H, pyridyl-2,6-b-H), 8.30 (s, 1H, fluorenyl-H), 8.10 (bs, 1H, fluorenyl-H), 8.02 (m, 1H, fluorenyl-H), 7.84 (bs, 1H, fluorenyl-H), 7.59 (d, 6H, J = 7.42, tosylate-2,6-H), 7.51 (m, 1H, fluorenyl-H), 7.37 (m, 1H, fluorenyl-H), 7.07 (d, J = 7.42, 6H, tosylate-3,5-H), 4.90 (s, 3H, N⁺-CH₃-a), 4.82 (s, 9H, N⁺-CH₃-b), 2.22 (s, 9H, tosylate-CH₃), 1.56 (s, 6H, fluorenyl-CH₃), -2.82 (s, 2H, pyrrole-NH)

**¹³C-NMR** (125 MHz, CD₃OD, 303.57 K, δ ):
158.9 (s, 3C, quart. ipso pyridyl-1-b-C), 158.9 (s, 1C, quart. ipso pyridyl-1-a-C), 156.7 (s, 1C, quart. fluorenyl-9a-C), 155.6 (s, 1C, quart. fluorenyl-8a-C), 149.2 (s, 8C, quart. α-pyrrole-C), 147.0 (d, 1C, pyridyl-4-a-CH), 147.0 (d, 1C, pyridyl-6-a-CH), 147 (d, 1C, pyridyl-2-a-CH), 145.7 (d, 6C, pyridyl-3,5-b-CH), 145.7 (d, 8C, β-pyrrole-CH), 143.4 (s, 1C, quart. fluorenyl-4a-C), 143.2 (s, 1C, quart. fluorenyl-4b-C), 143.2 (s, 3C, quart. tosylate-C), 141.6 (s, 3C, quart. tosylate-C), 134.3 (d, 6C, pyridyl-2,6-b-CH), 133.2 (s, 1C, quart. Pyridyl-5-a-C), 129.7 (d, 6C, tosylate-3,5-CH), 128.5 (d, 2C, fluorenyl-CH), 128.5 (d, 1C, fluorenyl-CH), 126.8 (d, 6C, tosylate-2,6-CH), 123.9 (d, 1C, fluorenyl-CH), 123.7 (d, 1C, fluorenyl-CH), 122.3 (d, 1C, fluorenyl-CH), 121.8 (d, 1C, fluorenyl-CH), 117.1 (s, 3C, quart. meso pyridyl-b-C), 117.1 (s, 1C, quart. ipso-fluorenyl-2-C), 115.4 (s, 1C, 1C, quart. meso pyridyl-a-C), 50.3 (s, 1C, quart. fluorenyl-9-C), 49.8 (q, 1C, N⁺-CH₃-a), 49.0 (q, 3C, N⁺-CH₃-b), 27.4 (q, 2C, fluorenyl-CH₃), 21.3 (q, 3C, tosylate-CH₃)

**ESI-MS** (Chloroform/Methanol 1:1, positive ion mode):
m/z = 290.07 ([M^{3+]}), m/z = 434.40 ([M²⁺], m/z = 606.20 ([M²⁺tos₂]), m/z = 520.27 ([M²⁺tos]), m/z = 347.13 ([M³⁺tos]), m/z = 217.80 ([M⁴⁺]).
C₅₉H₅₀N₈⁴⁺ theoretical mass = 871.08

### Example 9

### 5-(5-(2-(9,9-dimethyl)fluorenyl)-N-methylpyridinium-3-yl)-10,15,20-tris(N-methylpyridinium-4-yl)-21 H,23H-porphyrin chloride ("TMPy₃PyFluorenyl₁P-Cl₄")

6 µmol TMPy₃PyFluorenyl₁P-tos₄ are dissolved in 35 ml H₂O and 5 ml MeOH. Then 2 g of Dowex 1x8 Cl⁻ ion exchange resin are added. After 10 h of slow stirring, the ion exchange resin is filtered off and the remaining reaction mixture is evaporated to dryness, which gives the Cl⁻ salt of TMPy₃PyFluorenyl₁P⁴⁺. Yield: 98%

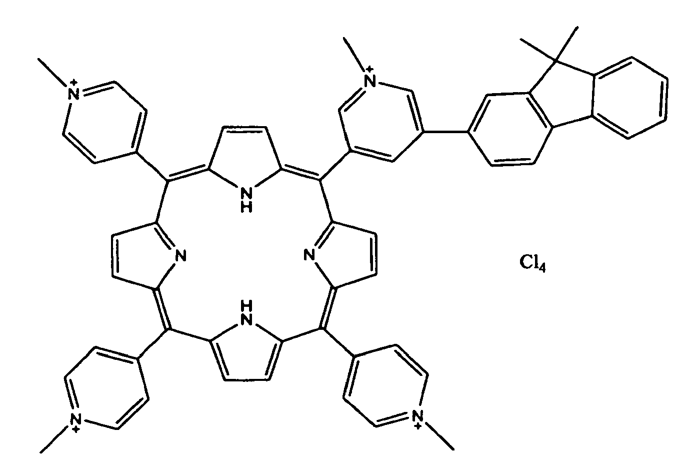

### Bis meso-N-methylpyridinium-3-yl substituted porphyrins modified with 9,9-dimethylfluorene

### Example 10

### Trans 5,10-bis(5-bromo-3-pyridyl)-15,20-bis(4-pyridyl)-21H,23H-porphyrin ("DPy₂PyBr₂P")

To a refluxing mixture of 50 ml propionic acid and 10 ml propionic anhydride 1.5 mmol 5-bromo-3-pyridinecarboxaldehyde, 4.5 mmol 4-pyridinecarboxaldehyde and 6.2 mmol of freshly distilled pyrrole are added. This mixture is refluxed for 150 min. Then reaction mixture is cooled to room temperature and 2 mmol of 2,3-dichloro-5,6-dicyano-1,4-benzoquinone (DDQ) are added. The mixture is stirred for 1 h, then the solvent is evaporated to dryness and the black-purple product is dissolved in CHCl₃. This mixture is divided into several parts and neutralized with a saturated NaHCO₃ water solution. The neutralized solution is dried over MgSO₄, filtered and evaporated to dryness. Due to the fact that during this statistical synthesis several porphyrin products are obtained, intensive column chromatography is needed to separate the different porphyrin species. Silica gel is used as stationary phase and the products are eluted with a mixture of CHCl₃ : MeOH 19:1 (v/v). The column chromatography is repeated several times with different mixtures of CHCl₃ MeOH v/v to finally obtain pure compounds. The desired derivative is chosen after mass spectrometer and NMR analysis of each obtained compound. *Yield 5* %

**¹H-NMR** (500 MHz, CDCl₃, 303.57 K, δ [ppm]):
9.37 (bs, 2H, pyridyl-4-a-H), 9.16 (bs, 2H, pyridyl-6-a-H), 9.07 (bs, 4H, pyridyl-3,5-b-H), 8.89 (s, 8H, β-pyrrole-H), 8.69 (s, 2H, pyridyl-2-a-H), 8.16 (bs, 4H, pyridyl-2,6-b-H), -2.90 (s, 2H, pyrrole-NH)

**¹³C-NMR** (125 MHz, CDCl₃, 303.57 K, δ [ppm]):
151.8 (d, 2C, pyridyl-4-a-CH), 150.8 (d, 2C, pyridyl-6-a-CH), 149.8 (s, 2C, quart. ipso pyridyl-1-C), 148.6 (d, 4C, pyridyl-3,5-b-CH), 146.8 (d, 8C, quart. β -pyrrole-C), 143.3 (d, 2C, pyridyl-2-a-CH), 139.1 (s, 2C, quart. ipso pyridyl-1-a-C), 131.8 (d, 8C, β-pyrrole-CH), 129.5 (d, 4C, pyridyl-2,6-CH), 120.0 (s, 2C, quart. meso pyridyl-a-C), 118.3 (s, 2C, quart. meso pyridyl-b-C), 115.2 (s, 2C, quart. pyridyl-3-a-C)

**ESI-MS** (Chloroform/Methanol 1:1, positive ion mode): m/z = 777.33 ([M+H]⁺), 779.33 ([M+H]⁺), 775.33 ([M+H]⁺)
C₄₀H₂₄Br₂N₈ theoretical mass = 776.05, 778.05, 774.05

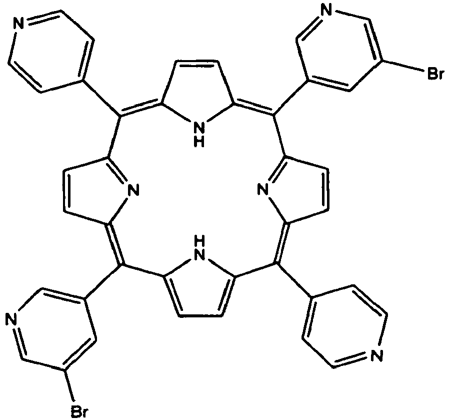

### Example 11

### Trans 5,10-bis(5-(2-(9,9-dimethyl)fluorenyl)-3-pyridyl)-15,20-bis(4-pyridyl)-21 H, 23H-porphyrin ("DPy₂PyFluorenyl₂P")

23.3 µmol DPy₂PyBr₂P and 49.6 µmol 9,9-dimethylfluorenyl-2-boronic acid dimethylester are dissolved in 40 ml toluene and 10 ml methanol. Then the mixture is degassed and saturated with argon. After that, 0.9 µmol tetrakis(triphenylphosphine)palladium(0) and 0.8 ml 2 M Na₂CO₃ solution are added under argon atmosphere. Then the reaction mixture is protected from light, heavily stirred and heated up to 80 °C for 67 h. After that, the reaction mixture is filtered, extracted with deionized water, dried over Na₂SO₄ and evaporated to dryness. For the purification of the crude product, intensive column chromatography is needed. Silica gel is used as stationary phase and the product is eluted using a mixture of CHCl₃ : MeOH 29:1 (v/v). The second band is the desired product, which is evaporated to dryness after the separation process. Yield: 82 %

**¹H-NMR** (500 MHz, CDCl₃, 303.57 K, δ ):
9.45 (m, 2H, pyridyl-4-a-H), 9.39 (d, J = 2.19, 2H, pyridyl-6-a-H), 9.08 (d, J = 5.53, 4H, pyridyl-3,5-b-H), 9.00 (d, J = 6.86, 4H, β-pyrrole-H), 8.91 (d, J = 6.86, 4H, β-pyrrole-H), 8.80 (m, 2H, pyridyl-2-a-H), 8.19 (d, J = 5.61, 4H, pyridyl-2,6-b-H), 7.88 (m, 4H, fluorenyl-3,4-H), 7.88 (s, 2H, fluorenyl-1-H), 7.77 (m, 2H, fluorenyl-5-H), 7.45 (m, 2H, fluorenyl-8-H), 7.36 (m, 4H, fluorenyl-6,7-H), 1.55 (s, 12H, fluorenyl-CH₃), -2.78 (s, 2H, pyrrole-NH)

**¹³C-NMR** (125 MHz, CDCl₃, 303.57 K, δ ):
154.7 (s, 2C, quart. fluorenyl-8a-C), 153.7 (s, 2C, quart. fluorenyl-9a-C), 151.8 (d, 2C, pyridyl-4-a-CH), 149.7 (s, 2C, quart. ipso pyridyl-1-b-C), 148.3 (d, 4C, pyridyl-3,5-b-CH), 147.7 (d, 2C, pyridyl-6-a-CH), 146.9 (s, 8C, quart. α -pyrrole-C), 139.7 (s, 2C, quart. fluorene-4a, 4b-C), 139.0 (d, 2C, pyridyl-2-a-CH), 137.3 (s, 1C, quart. ipso pyridyl-1-a-C), 135.9 (s, 2C, quart. pyridyl-5-a-C), 135.2 (d, 2C, quart. ipso-fluorenyl-2-C), 131.8 (d, 8C, β-pyrrole-CH), 129.1 (d, 4C, pyridyl-2,6-b-CH), 127.0 (d, 4C, fluorenyl-6,7-CH), 126.4 (d, 2C, fluorenyl-1-CH), 122.5 (d, 2C, fluorenyl-8-CH), 121.5 (d, 4C, fluorenyl-3,4-CH), 120.7 (d, 2C, fluorenyl-5-CH), 117.5 (s, 2C, quart. meso pyridyl-b-C), 116.8 (s, 2C, quart. meso pyridyl-a-C), 47.0 (s, 2C, quart. fluorenyl-9-C), 27.0 (q, 4C, fluorenyl-CH₃)

**ESI-MS** (Chloroform/Methanol 1:1, positive ion mode): m/z = 1003.61 ([M+H]⁺), C₇₀H₅₀N₈ theoretical mass = 1002.42

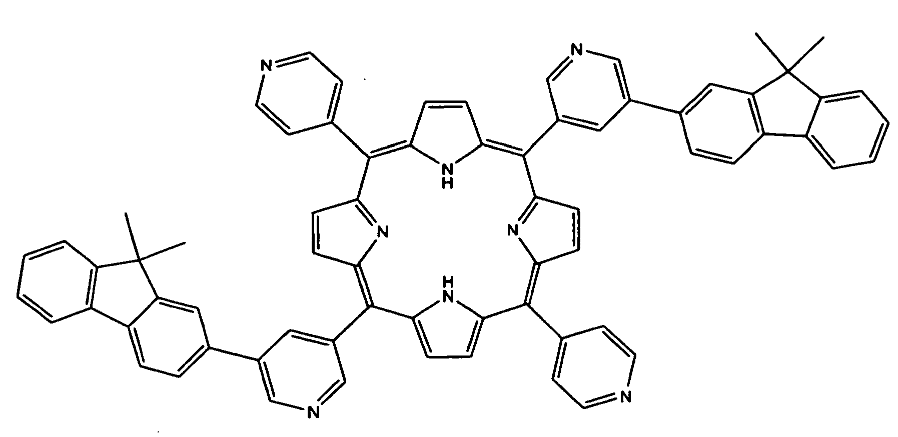

### Example 12

### Trans 5,10-bis(5-(2-(9,9-dimethyl)fluorenyl)-N-methylpyridinium-3-yl)-bis-15,20-(N-methylpyridinium-4-yl)-21H,23H-porphyrin chloride ("DMPy₂PyFluorenyl₂P-Cl₄")

To a mixture of 12 ml CH₃NO₂ and 12 ml CHCl₃ (1:1 v/v) 7.2 µmol DPy₂PyFluorenyl₂P, and 0.72 mmol methyl p-toluene-sulfonate are added. This reaction mixture is refluxed for 72 h under argon atmosphere. The progress of the N-methylation reaction can be monitored using silica gel TLC and an eluent containing ACN : H₂O(sat. KNO₃) : H₂O 8:1:1 (v/v). Then the reaction mixture is evaporated to dryness, dissolved in deionized water and the impurities are extracted with CHCl₃. The remaining water fraction is filtered and then evaporated to dryness, which gives the tosylate salt of DMPy₂PyFluorenyl₂P⁴⁺.

DMPy₂PyFluorenyl₂P-tos₄ is dissolved in 35 ml H₂O and 5 ml MeOH. Then 2 g of Dowex 1 x8 CI⁻ ion exchange resin are added. After 10 h of slow stirring, the ion exchange resin is filtered off and the remaining reaction mixture is evaporated to dryness, which gives the Cl- salt of DMPy₂PyFluorenyl₂P⁴⁺. *Yield.* 90%

**¹H-NMR** (500 MHz, CDCl₃, 303.57 K, δ ):
9.94 (s, 2H, pyridyl-4-a-H), 9.91 (s, 2H, pyridyl-6-a-H); 9.76 (s, 2H, pyridyl-2-aH), 9.43 (bs, 4H, pyridyl-3,5-b-H), 9.36 (m, 4H, β-pyrrole-H), 9.23 (m, 4H, β -pyrrole-H), 9.01 (bs, 4H, pyridyl-2,6-b-H), 8.30 (s, 2H, fluorenyl-1-H), 8.15 (m, 2H, fluorenyl-3-H), 8.12 (m, 2H, fluorenyl-4-H), 7.85 (m, 2H, fluorenyl-5-H), 7.52 (m, 2H, fluorenyl-8-H), 7.38 (m, 4H, fluorenyl-6,7-H), 4.90 (s, 6H, N⁺-CH₃-a), 4.84 (s, 6H, N⁺-CH₃-b), 1.57 (s, 12H, fluorenyl-CH₃), -2.87 (s, 2H, pyrrole-NH)

**¹³C-NMR** (125 MHz, CDCl₃, 303.57 K, δ ):
159.1 (s, 2C, quart. ipso pyridyl-1-b-C), 156.7 (s, 2C, quart. fluorenyl-8a-C), 155.6 (s, 2C, quart. fluorenyl-9a-C), 149.1 (s, 8C, quart. α-pyrrole-C), 148.4 (s, 2C, quart. ipso pyridyl-1-a-C), 147.0 (d, 2C, pyridyl-6-a-CH), 147.0 (d, 2C, pyridyl-2-a-CH), 145.7 (d, 4C, pyridyl-3,5-b-CH), 145.0 (d, 2C, pyridyl-4-a-CH), 143.3 (s, 2C, quart. fluorenyl-4a-C), 142.6 (s, 1C, quart. fluorenyl-4b-C), 141.5 (s, 2C, quart. pyridyl-5-a-C), 134.3 (d, 4C, pyridyl-2,6-CH), 133.8 (d, 8C, β -pyrrole-CH), 133.2 (d, 2C, quart. ipso fluorenyl-2-C), 131.4 (d, 2C, fluorenyl-3-CH), 128.5 (d, 4C, fluorenyl-6,7-CH), 128.5 (d, 2C, fluorenyl-4-CH), 123.9 (d, 2C, fluorenyl-8-CH), 123.7 (d, 2C, fluorenyl-1-CH), 121.8 (d, 2C, fluorenyl-5-CH), 116.8 (s, 2C, quart. meso pyridyl-b-C), 115.2 (s, 2C, quart. meso pyridyl-a-C), 49.5 (q, 2C, N⁺-CH₃-a), 48.9 (q, 2C, N⁺-CH₃-b), 48.1 (s, 2C, quart. fluorenyl-9-C), 27.4 (q, 4C, fluorenyl-CH₃)

**ESI-MS** (Methanol 1:1, positive ion mode): m/z = 265.91 ([M]⁴⁺), 354.26 ([M]³⁺), 530.56 ([M]²⁺)
C₇₄H₆₂N₈ ⁴⁺ theoretical mass = 1062.51

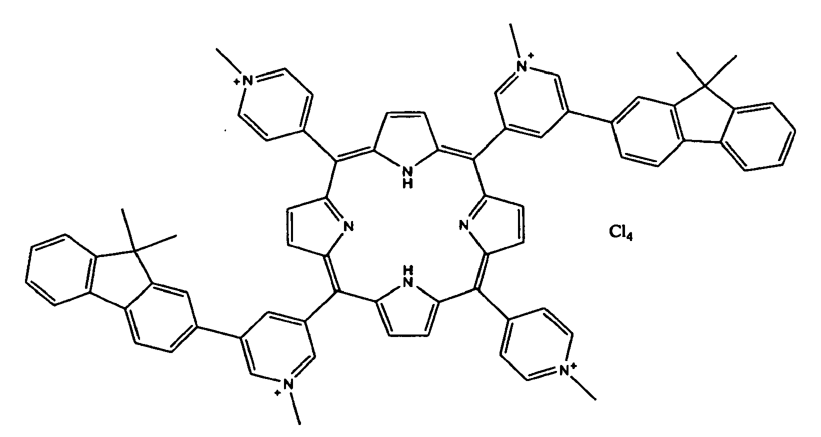

### Example 13

### Synthesis of 5,10,15-tris(5-bromo-3-pyridyl)-20-(1-pyrenyl)-21H,23H-porphyrin ("TPyBr₃Pyr₁P")

To a refluxing mixture of 50 ml propionic acid and 1 ml propionic anhydride 1.5 mmol 1-pyrenecarboxaldehyde, 4.5 mmol 5-bromo-3-pyridinecarboxaldehyde and 6.2 mmol of freshly distilled pyrrole are added. This mixture is refluxed for 110 min. Then the solvent is evaporated to dryness and the black-purple product is dissolved in CHCl₃. The black-purple mixture is divided into several parts and neutralized with saturated NaHCO₃ water solution. The neutralized solution is dried over MgSO₄, filtered and evaporated to dryness. During the synthesis of asymmetrically substituted porphyrins several porphyrin species are obtained. Therefore, intensive column chromatography is needed to separate the different porphyrin species. Silica gel is used as stationary phase and the products are eluted with a mixture of CHCl₃ : MeOH 19:1 (v/v) and CHCl₃ MeOH 49:1 (v/v). The desired crude product is the fifth fraction on the column. The column chromatography is repeated several times with different mixtures of CHCl₃ MeOH (v/v) to finally obtain pure compounds. The desired compound is chosen, after mass spectrometer and NMR analysis of each obtained compound. *Yield.* 7 %

**¹H-NMR** (500 MHz, CDCl₃, 303.57 K, δ ):
9.40 (s, 1H, pyridyl-4-H-b), 9.37 (s, 2H, pyridyl-4-H-a), 9.17 (d, J = 1.95, 1H, pyridyl-6-H-b), 9.11 (d, J = 1.95, 2H, pyridyl-6-H-a), 8.89 (s, 4H, β-pyrrole-H), 8.78 (bs, 1H, pyrenyl-H), 8.73 (s, 1H, Pyridyl-2-H-b), 8.70 (s, 1H, pyridyl-2-H-b), 8.70 (bs, 2H, □-pyrrole-H), 8.61 (bs, 2H, β-pyrrole-H), 8.53 (d, J = 7.30, 1H, pyrenyl-H), 8.39 (d, J = 8.93, 1H, pyrenyl-H), 8.33 (d, J = 7.75, 1H, pyrenyl-H), 8.31 (d, J = 9.24, 1H, pyrenyl-H), 8.10 (d, J = 7.75, 1H, pyrenyl-H), 8.06 (d, J = 7.30, 1H, pyrenyl-H), 7.70 (d, J = 8.93, 1H, pyrenyl-H), 7.38 (m, 1H, pyrenyl-H), -2.64 (s, 2H, pyrrole-NH)

¹³**C-NMR** (125 MHz, CDCl₃, 303.57 K, δ ):
151.7 (d, 1C, pyridyl-4-b-CH), 151.7 (d, 2C, pyridyl-4-a-CH), 150.6 (d, 1C, pyridyl-6-b-CH), 150.6 (d, 2C, pyridyl-6-a-CH), 146.6 (s, 8C, quart. α-pyrrole-C), 143.3 (d, 1C, pyridyl-2-b-CH), 143.3 (d, 2C, pyridyl-2-a-CH), 139.1 (s, 3C, quart. ipso pyridyl-1-C), 139.1 (s, 1C, quart. ipso pyrenyl-1-C), 135.9 (s, 1C, quart. pyrenyl-C), 133.7 (s, 1C, quart. pyrenyl-C), 132.7 (d, 1C, pyrenyl-CH), 132.7 (d, 2C, β-pyrrole-CH), 131.9 (s, 1C, quart. pyrenyl-C), 131.3 (d, 6C, β-pyrrole-CH), 130.8 (s, 1C, quart. pyrenyl-C), 128.5 (d, 1C, pyrenyl-CH), 128.2 (d, 1C, pyrenyl-CH), 127.7 (d, 1C, pyrenyl-CH), 126.8 (d, 1C, pyrenyl-CH), 126.6 (d, 1C, pyrenyl-CH), 126.0 (d, 1C, pyrenyl-CH), 125.6 (d, 1C, pyrenyl-CH), 124.6 (s, 1C, quart. pyrenyl-C), 124.3 (s, 1C, quart. pyrenyl-C), 123.0 (d, 1C, pyrenyl-CH), 119.9 (s, 3C, quart. pyridyl-5-b-C), 117.5 (s, 1C, quart. meso pyrenyl-C), 115.1 (s, 2C, quart. meso pyridyl-a-C), 114.9 (s, 1C, quart. meso pyridyl-b-C)

**ESI-MS** (Chloroform/Methanol 1:1, positive ion mode): m/z = 979.84 ([M+H]⁺). C₅₁H₂₈Br₃N₇ theoretical mass = 976.99, 978.99, 977.99

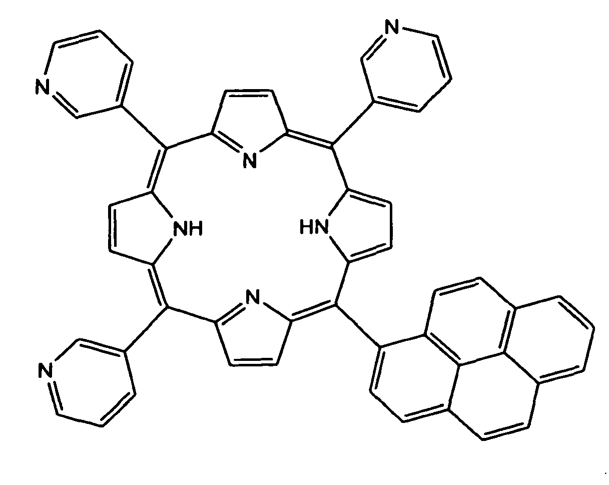

### Example 14

### Synthesis of 5,10,15-tris-(5-(3-(N-phenylsulfonyl-)indolyl)-3-pyridyl)-20-(1-pyrenyl)-21H, 23H-porphyrin ("TPylndolprot₃Pyr₁P")

14.3 µmol TPyBr₃Pyr₁P and 85.8 µmol 1-(phenylsulfonyl)-3-indoleboronic acid are dissolved in 40 ml toluene and 10 ml methanol. Then the mixture is degassed and saturated with argon. After that, 1.2 mmol tetrakis(triphenylphosphine)palladium(0) and 1 ml 2 M Na₂CO₃ solution are added under argon atmosphere. Then the reaction mixture is protected from light, heavily stirred and heated up to 80 °C for 30 h. After that, the reaction mixture is filtered, extracted with deionized water, dried over Na₂SO₄ and evaporated to dryness. For the purification of the crude product, intensive column chromatography is needed. Silica gel is used as stationary phase and the product is eluted using a mixture of CHCl₃ MeOH 49:1 (v/v). The second band is the desired product, which is evaporated to dryness after the separation process. *Yield.* 79 %

**¹H-NMR** (500 MHz, CDCl₃, 303.57 K, δ ):
9.48 (s, 1 H, pyridyl-4-H-b), 9.48 (s, 1H, pyridyl-4-H-a), 9.34 (s, 1H, pyridyl-6-H-b), 9.34 (s, 1H, pyridyl-6-H-a), 9.00 (s, 4H, β-pyrrole-H), 8.80 (bs, 1H, pyrenyl-H), 8.80 (bs, 2H, β-pyrrole-H), 8.80 (s, 1H, pyridyl-2-H-a), 8.80 (s, 1H, pyridyl-2-H-b), 8.63 (bs, 2H, β-pyrrole-H), 8.56 (bs, 1H, pyrenyl-H), 8.41 (bs, 1H, pyrenyl-H), 8.36 (bs, 1H, pyrenyl-H), 8.34 (bs, 1H, pyrenyl-H), 8.12 (bs, 1H, pyrenyl-H), 8.10 (bs, 3H, indolyl-7-H), 8.08 (bs, 1H, pyrenyl-H), 7.98 (s, 3H, indolyl-3-H), 7.97 (bs, 6H, phenylsulfonyl-2,6-H), 7.93 (d, 3H, indol-4-H), 7.72 (bs, 1H, pyrenyl-H), 7.53 (m, 3H, phenylsulfonyl-4-H), 7.45 (m, 6H, phenylsulfonyl-2,6-H), 7.44 (bs, 1H, pyrenyl-H), 7.40 (m, 3H, indolyl-6-H), 7.29 (m, 3H, indolyl-5-H), -2.52 (s, 2H, pyrrole-NH)

**¹³C-NMR** (125 MHz, CDCl₃, 303.57 K, 8):
152.3 (d, 1C, pyridyl-4-b-CH), 152.3 (d, 2C, pyridyl-4-a-CH), 148.0 (d, 1C, pyridyl-6-b-CH), 148.0 (d, 2C, pyridyl-6-a-CH), 146.3 (d, 3C, quart. ispo pyridyl-1-C), 146.0 (s, 1C, quart. ipso pyrenyl-1-C), 142.3 (s, 8C, quart. α-pyrrole-C), 140.0 (d, 1C, pyridyl-2-b-CH), 140.0 (d, 2C, pyridyl-2-a-CH), 138.2 (s, 4C, quart. indolyl-b-C), 138.0 (s, 2C, quart. indolyl-a-C), 137.6 (d, 2C, quart. pyridyl-5-b-CH), 137.3 (s, 1C, quart. pyridyl-5-a-CH), 136.2 (s, 1C, quart. pyrenyl-C), 135.0 (s, 3C, quart. phenylsulfonyl-1-C), 134.3 (d, 3C, phenylsulfonyl-4-CH), 134.0 (s, 1C, quart. pyrenyl-C), 132.8 (d, 4C, β-pyrrole-CH), 131.9 (s, 1C, quart. pyrenyl-C), 131.3 (d, 1C, pyrenyl-CH), 131.3 (d, 4C, β-pyrrole-CH), 130.9 (s, 1C, quart. pyrenyl-C), 129.6 (d, 6C, phenylsufonyl-3,5-CH), 128.9 (s, 3C, quart. indolyl-C), 128.6 (d, 1C, pyrenyl-CH), 128.3 (d, 1C, pyrenyl-CH), 127.4 (d, 1C, pyrenyl-CH), 127.1 (d, 6C, phenylsulfonyl-2,6-CH), 127.1 (d, 1C, pyrenyl-CH), 126.6 (d, 1C, pyrenyl-CH), 126.6 (d, 1C, pyrenyl-CH), 126.0 (d, 1C, pyrenyl-CH), 126.0 (d, 1C, pyrenyl-CH), 125.7 (d, 3C, indolyl-6-CH), 124.7 (s, 1C, quart. pyrenyl-C), 124.3 (s, 1C, quart. pyrenyl-C), 124.3 (d, 3C, indolyl-3-CH), 124.3 (d, 3C, indolyl-5-CH), 122.7 (d, 1C, pyrenyl-CH), 120.2 (d, 3C, indolyl-4-CH), 119.8 (s, 1C, quart. meso pyrenyl-C), 116.3 (s, 2C, quart. meso pyridyl-a-C), 116.1 (s, 1C, quart. meso pyridyl-b-C), 114.2 (d, 3C, indolyl-7-CH)

**ESI-MS** (Chloroform/Methanol 1:1, positive ion mode): m/z = 1507.07 ([M+H]⁺). C₉₃H₅₈N₁₀O₆S₃ theoretical mass = 1506.37

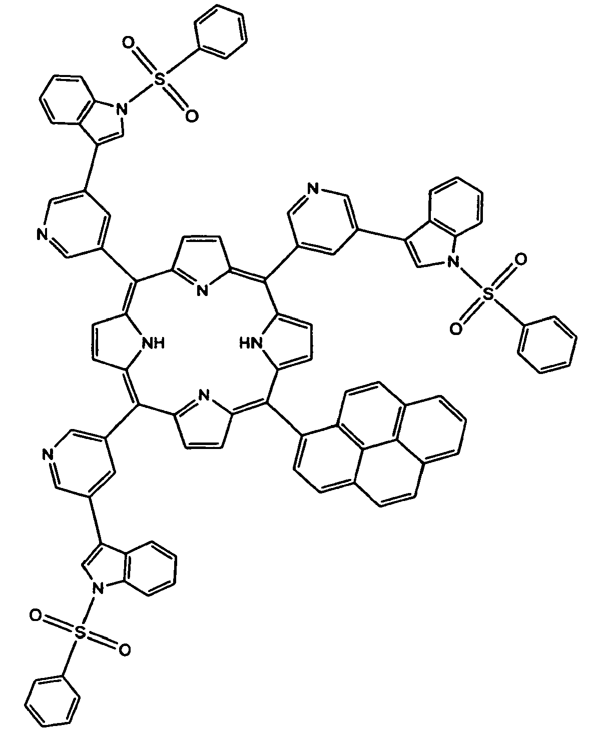

### Example 15

### Synthesis of 5,10,15-tris(5-(3-(N-phenylsulfonyl-)indolyl)-N-methyl-pyridinium-3-yl)-20-(1-pyrenyl)-21H,23H-porphyrin chloride ("TMPylndolprot₃Pyr₁P-Cl₃")

To a mixture of 15 ml CH₃NO₂ and 15 ml CHCl₃ (1:1 v/v) 1.9 µmol TPylndolPhen₃Pyr₁P, and 0.142 mmol methyl p-toluene-sulfonate are added. This reaction mixture is refluxed for 88 h under argon atmosphere. The progress of the N-methylation reaction can be monitored using silica gel TLC and an eluent containing ACN : H₂O(sat. KNO₃) : H₂O 8:1:1 (v/v).

Then the reaction mixture is evaporated to dryness, dissolved in deionized water and the impurities are extracted with CHCl₃. The remaining water fraction is filtered and then evaporated to dryness, which gives the tosylate salt of TMPylndolPhen₃Pyr, P³⁺.

TMPylndolPhen₃Pyr₁P-tos₃ is dissolved in 35 ml H₂O and 5 ml MeOH. Then 2 g of Dowex 1x8 Cl⁻ ion exchange resin are added. After 10 h of slow stirring, the ion exchange resin is filtered off and the remaining reaction mixture is evaporated to dryness, which gives the Cl⁻ salt of TMPylndolPhen₃Pyr₁P³⁺. *Yield:* 89 %

**¹H-NMR (**500 MHz, CDCl₃, 303.57 K, δ ):
Assignment could not be done due to peak broadening

**¹³C-NMR** (125 MHz, CDCl₃, 303.57 K, δ ):
Assignment could not be done due insufficient information from the ¹H spectra

**ESI-MS** (Methanol 1:1, positive ion mode): m/z = 517.59 ([M]³⁺), 775.93 ([M]²⁺), 861.87 [M+toS]²⁺. C₉₆H₆₇N₁₀O₆S₃³⁺ theoretical mass = 1551.44

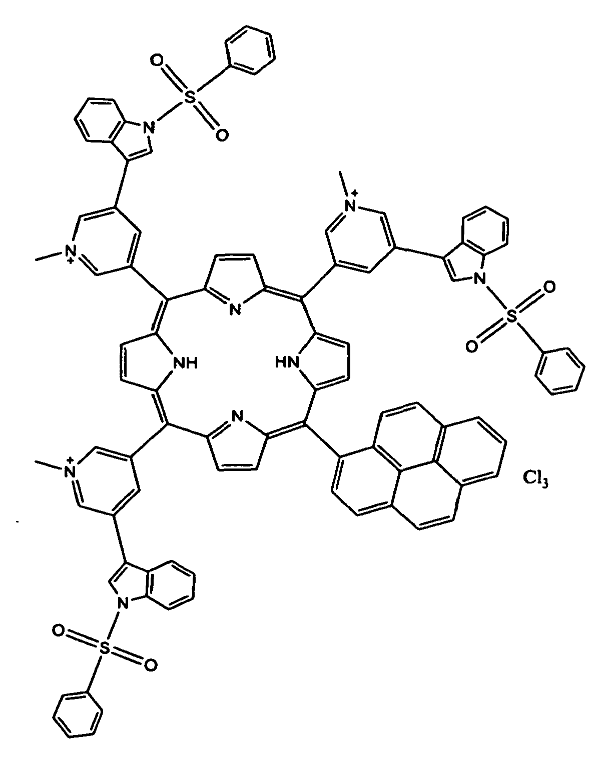

### Example 16

### Synthesis of 5,10,15-tris(4-pyridyl)-20-(1-pyrenyl)-21H,23H-porphyrin ("TPy₃Pyr₁P")

To a refluxing mixture of 50 ml propionic acid and 1 ml propionic anhydride 1.5 mmol 1-pyrenecarboxaldehyde, 4.5 mmol 4-pyridinecarboxaldehyde and 6.2 mmol of freshly distilled pyrrole are added. This mixture is refluxed for 90 min. Then the solvent is evaporated to dryness and the black-purple product is dissolved in CHCl₃. This mixture is divided into several parts and neutralized with a saturated NaHCO₃ water solution. The neutralized solution is dried over MgSO₄, filtered and evaporated to dryness. Due to the fact that during this asymmetric synthesis several porphyrin products are obtained, intensive column chromatography is needed to separate the different porphyrin species. Silica gel is used as stationary phase and the products are eluted with a mixture of CHCl₃ MeOH 19:1 (v/v). The column chromatography is repeated several times with different mixtures of CHCl₃ MeOH v/v to finally obtain pure compounds. The desired compound is chosen, after mass spectrometer and NMR analysis of each obtained compound. For further purification the porphyrin compounds are recrystallized using MeOH. *Yield:* 9 %

**¹H-NMR** (500 MHz, CDCl₃, 303.57 K, δ ):
9.08 (d, J = 4.62, 2H, pyridyl-3,5-H), 9.02 (d, J = 4.53, 4H, pyridyl-3,5-H), 8.88 (s, 4H, β-pyrrole-H), 8.78 (d, J = 7.52, 1H, pyrenyl-H), 8.72 (d, J = 4.26, 2H, β -pyrrole-H), 8.58 (d, J = 4.26, 2H, β-pyrrole-H), 8.53 (d, J = 7.52, 1H, pyrenyl-H), 8.40 (d, J = 8.88, 1H, pyrenyl-H), 8.34 (t, J = 8.22, 2H, pyrenyl-H), 8.20 (d, J = 4.62, 2H, pyridyl-2,6-H), 8.16 (bs, 4H, pyridyl-2,6-H), 8.08 (m, 2H, pyrenyl-H), 7.70 (d, J = 9.33, 1H, pyrenyl-H), 7.38 (d, J = 9.33, 1H, pyrenyl-H), -2.64 (s, 2H, pyrrole-NH)

**¹³C-NMR** (125 MHz, CDCl₃, 303.57 K, δ ):
(s = C, d = CH₂, t = CH₃)
162.5 (s, 1C, quart. ipso pyrenyl-1-C), 150.0 (s, 3C, quart. ipso pyridyl-1-C), 148.6 (d, 2C, pyridyl-3,5-a-CH), 148.5 (d, 4C, pyridyl-3,5-b-CH), 136.2 (s 2C, quart. pyrenyl-C), 133.7 (s, 2C, quart. pyrenyl-C), 132.7 (d, 1C, pyrenyl-CH), 132.7 (d, 2C, β-pyrrole-CH), 131.9 (s, 3C, α-pyrrole-C), 131.9 (s, 3C, α -pyrrole-C), 131.3 (d, 2C, β-pyrrole-CH), 131.2 (d, 4C, β-pyrrole-CH), 130.9 (s, 2C, α -pyrrole-C), 129.5 (d, 2C, pyridyl-2,6-CH), 129.5 (d, 4C, pyridyl-2,6-CH), 128.5 (d, 1C, pyrenyl-CH), 128.1 (d, 1C, pyrenyl-CH), 127.8 (d, 1C, pyrenyl-CH), 126.9 (d, 1C, pyrenyl-CH), 126.6 (d, 1C, pyrenyl-CH), 126.0 (d, 1C, pyrenyl-CH), 125.6 (d, 1C, pyrenyl-CH), 124.7 (s, 1C, quart. pyrenyl-C), 124.3 (s, 1C, quart. pyrenyl-C), 123.0 (d, 1C, pyrenyl-CH), 119.6 (s, 1C, quart. meso pyrenyl-C), 117.7 (s, 1C, quart. meso pyridyl-a-C), 117.6 (s, 1C, quart. meso pyridyl-b-C)

**ESI-MS** (Chloroform/Methanol 1:1, positive ion mode): m/z = 742.60 ([M+H]⁺). C₅₁H₃₁N₇ theoretical mass = 741.26

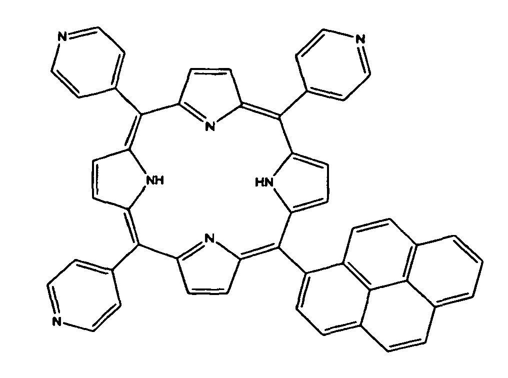

### Example 17

### Synthesis of 5,10,15-tris(N-methylpyridinium-4-yl)-20-(1-pyrenyl)-21 H,23H-porphyrin tosylate ("TMPy₃Pyr₁P-tos₃")

To a mixture of 16 ml CH₃NO₂ and 8 ml CHCl₃ (2:1 v/v) 24 µmol TPy₃Pyr₁P, and 1.4 mmol methyl p-toluene-sulfonate is added. This reaction mixture is refluxed for 60 h under argon atmosphere. The progress of the N-methylation reaction can be monitored using silica gel TLC and an eluent containing ACN : H₂O (sat. KNO₃) : H₂O 8:1:1 (v/v).Then the reaction mixture is evaporated to dryness, dissolved in deionized water and the impurities are extracted with CHCl₃. The remaining water fraction is filtered and evaporated to dryness, which gives the tosylate salt of TMPy₃Pyr₁P³⁺. *Yield:* 93 %

**¹H-NMR** (500 MHz, CD₃OD, 303.57 K, δ ):
9.35 (d, J = 5.48, 2H, pyridyl-3,5-H), 9.21 (d, J = 5.45, 4H, pyridyl-2,6-H), 9.11 (bs, 4H, β-pyrrole-H), 8.91 (d, J = 5.48, 2H, pyridyl-3,5-H), 8.73 (bs, 4H, pyridyl-2,6-H), 8.73 (bs, 2H, β-pyrrole-H), 8.50 (d, J = 7.35, 1H, pyrenyl-H), 8.50 (bs, 2H, β-pyrrole-H), 8.18 (d, J = 7.35, 1H, pyrenyl-H), 8.01 (m, 1H, pyrenyl-H), 7.99 (m, 2H, pyrenyl-H), 7.61 (m, 1H, pyrenyl-H), 7.60 (d, J = 7.92, 12H, 3x tosylate-3,5-H), 7.51 (d, J = 6.67, 1H, pyrenyl-H), 7.24 (d, J = 9.20, 1H, pyrenyl-H), 7.05 (d, J = 9.20, 1H, pyrenyl-H), 6.98 (d, J = 7.92, 12 H, 3x tosylate-2,6-H), 4.78 (s, 3H, N⁺-CH₃), 4.68 (s, 6H, N⁺-CH₃), 2.08 (s, 9 H, 3x tosylate CH₃), -2.74 (s, 2H, pyrrole-NH)

**¹³C-NMR** (125 MHz, CD₃OD, 303.57 K, δ ):
159.5 (s, 1C, quart. ipso pyrenyl-1-C), 159.3 (s, 3C, quart. ipso-pyridyl-1-C), 145.5 (d, 2C, pyridyl-3,5-a-CH), 145.3 (d, 4C, pyridyl-3,5-b-CH), 143.6 (s, 3C, quart. tosylate-1-C), 141.5 (s, 3C, quart. tosylate-4-C), 136.2 (s, 2C, quart. pyrenyl-C), 134.5 (s, 3C, quart. α-pyrrole-C), 134.1 (d, 2C, pyridyl-2,6-CH), 133.9 (d, 4C, pyridyl-2,6-CH) 133.9 (d, 2C, β-pyrrole-CH), 133.5 (d, 1C, Pyrenyl-CH), 133.5 (d, 2C, β-pyrrole-CH), 133.2 (d, 4C, β-pyrrole-CH), 133.0 (s, 3C, α-pyrrole-C), 132.6 (s, 2C, α-pyrrole-C), 131.4 (s, 1C, quart. pyrenyl-C), 129.6 (d, 6C, tosylate-2,6-CH), 128.9 (d, 1C, pyrenyl-CH), 128.3 (d, 1C, pyrenyl-CH), 127.3 (d, 1C, pyrenyl-CH), 127.0 (d, 1C, pyrenyl-CH), 127.0 (d, 1C, pyrenyl-CH), 126.8 (d, 6C, tosylate-3,5-CH), 126.2 (d, 1C, pyrenyl-CH), 125.0 (s, 1C, quart. pyrenyl-C), 124.9 (s, 1C, quart. pyrenyl-C), 123.9 (d, 1C, pyrenyl-CH), 122.8 (s, 1C, quart. pyrenyl-1-C), 122.8 (s, 1C, quart meso pyrenyl-C), 116.3 (s, 1C, quart. meso pyridyl-C), 115.8 (s, 1C, quart. meso pyridyl-C), 49.8 (q, 1C, N⁺-CH₃), 49.6 (q, 2C, N⁺-CH₃), 21.1 (q, 1C, tosylate-CH₃)

**ESI-MS** (Methanol, positive ion mode): m/z = 262.20 ([M]³⁺), 392.80 ([M]²⁺), 478.60 ([M+tos]²⁺)
C₅₄H₄₀N₇³⁺ theoretical mass = 786.33

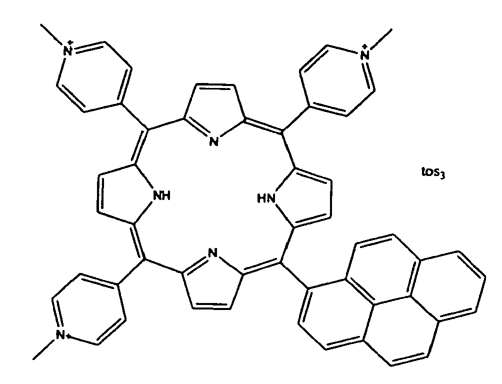

### Example 18

### Synthesis of 5,10,15-tris(N-ethylpyridinium-4-yl)-20-(1-pyrenyl)-21H,23H-porphyrin tosylate ("TEPy₃Pyr₁P-tos₃")

To a mixture of 15 ml CH₃NO₂ and 15 ml CHCl₃ (1:1 v/v) 14 µmol TPy₃Pyr₁P (1), and 1.1 mmol ethyl p-toluene-sulfonate are added. This reaction mixture is refluxed for 120 h under argon atmosphere. The progress of the N-ethylation reaction can be monitored using silica gel TLC and an eluent containing ACN :

H₂O (sat. KNO₃) H₂O 8:1:1 (v/v).Then the reaction mixture is evaporated to dryness and dissolved in deionized water and the impurities are extracted with CHCl₃. The remaining water fraction is filtered and then evaporated to dryness, which gives the tosylate salt of TEPy₃Pyr₁P³⁺. *Yield:* 96 %

**¹H-NMR** (500 MHz, CD₃OD, 303.57 K, δ ):
9.46 (d, J = 5.68, 2H, pyridyl-3,5-H-a), 9.32 (d, J = 4.48, 4H, pyridyl-3,5-H-b), 9.13 (s, 4H, β-pyrrole-H), 8.95 (d, J = 5.68, 2H, pyridyl-2,6-H-a), 8.82 (bs, 2H, β -pyrrole-H), 8.78 (d, J = 4.48, 4H, pyridyl-2.6-H-b), 8.52 (d, J = 7.50, 1H, pyrenyl-H), 8.48 (bs, 2H, β-pyrrole-H), 8.20 (d, J = 7.48, 1H, pyrenyl-H), 8.02 (m, 3H, pyrenyl-H), 7.65 (d, J = 7.50, 1H, pyrenyl-H), 7.61 (d, J = 7.89, 6H, tosylate-2,6-H), 7.54 (d, J = 7.06, 1H, pyrenyl-H), 7.28 (d, J = 9.29, 1H, pyrenyl-H), 7.06 (d, J = 9.29, 1H, pyrenyl-H), 6.99 (d, J = 7.89, 6H, tosylate-3,5-H), 5.06 (q, J = 7.28, 2H, N⁺-CH₂-a), 4.95 (q, J = 7.22, 2H, N⁺-CH₂-b), 2.08 (s, 9H, tosylate-CH₃), 1.98 (t, J = 7.28, 2H, N⁺-R-CH₃-a), 1.88 (t, J = 7.22, 2H, N⁺-R-CH₃-b), -2.74 (s, 2H, pyrrole-NH)

**¹³C-NMR** (125 MHz, CD₃OD, 303.57 K, δ ):
159.7 (s, 1C, quart. ipso pyrenyl-1-C), 159.5 (s, 3C, quart. ipso pyridyl-1-C), 150.3 (s, 8C, quart. α-pyrrole), 144.4 (d, 2C, pyridyl-3,5-CH-a), 144.2 (d, 4C, pyridyl-3,5-CH-b), 143.5 (s, 6C, quart. tosylate-1-C), 141.5 (s, 6C, quart. tosylate-4-C), 136.3 (s, 1C, quart. pyrenyl-C), 134.5 (s, 1C, quart. pyrenyl-C), 134.5. (d, 2C, pyridyl-2,6-CH-a), 134.3 (d, 4H, pyridyl-2,6-CH-b), 133.5 (d, 8C, β -pyrrole-CH), 133.5 (d, 1C, pyrenyl-CH), 133.1 (s, 1C, quart. pyrenyl-C), 132.7 (s, 3C, quart. tosylate-4-C), 131.4 (s, 1C, quart. pyrenyl-C), 129.7 (d, 6C, tosylate-2,6-CH), 129.4 (d, 1C, pyrenyl-CH), 129.0 (d, 1C, pyrenyl-CH), 128.3 (d, 1C, pyrenyl-CH), 127.3 (d, 1H, pyrenyl-CH), 127.0 (d, 1H, pyrenyl-CH), 126.8 (d, 6C, tosylate-3,5-CH), 126.6 (d, 1C, pyrenyl-CH), 126.3 (d, 1C, pyrenyl-CH), 125.1 (s, 1C, quart. pyrenyl-C), 124.9 (s, 1C, quart. pyrenyl-C), 123.9 (d, 1C, pyrenyl-CH), 122.8 (s, 1C, quart. meso pyrenyl-C), 116.4 (s, 1C, quart. meso pyridyl-C-a), 115.9 (s, 2C, quart. meso pyridyl-C-b), 58.6 (q, 1C, N⁺-CH₂-a), 58.5 (q, 1C, N⁺-CH₂-b), 21.1 (q, 9C, tosylate-CH₃), 16.8 (t,1C, N⁺-R-CH₂-a), 16.7 (t, 1C, N⁺-R-CH₂-b)

**ESI-MS** (Methanol, positive ion mode): m/z = 276.20 ([M]³⁺), 413.87 ([M]²⁺), 499.67 ([M+tos]²⁺), 1170.80 ([M+tos2]⁺)
C₅₇H₄₆N₇³+ theoretical mass = 829.02

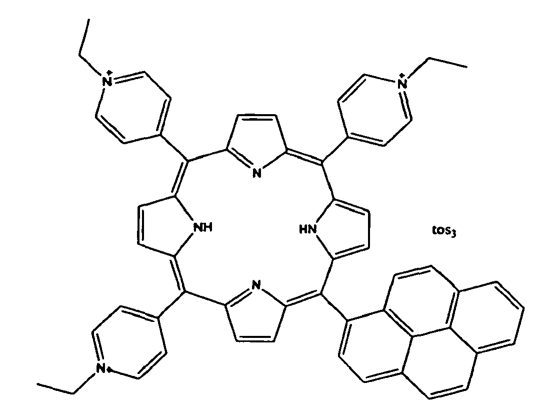

### Mono-substituted meso-6-bromo-indolyl porphyrins

### Example 19

### Synthesis of 5,10,15-tris(4-pyridyl)-20-(3-(6-bromo)-N-propionyl)-indolyl)-21 H,23H-porphyrin ("TPy31ndolBrN-Propl P")

To a refluxing mixture of 42 ml propionic acid and 12 ml propionic anhydride 1.5 mmol 6-bromo-indole-3-carboxaldehyde, 4.5 mmol 4-pyridinecarboxaldehyde and 6.2 mmol of freshly distilled pyrrole are added. This mixture is refluxed for 120 min. Then the solvent is evaporated to dryness and the black-purple product is dissolved in CHCl₃. This mixture is divided into several parts and neutralized with a saturated NaHCO₃ water solution. The neutralized solution is dried over MgSO₄, filtered and evaporated to dryness. Due to the fact that during this statistical synthesis several porphyrin products are obtained, intensive column chromatography is needed to separate the different porphyrin species. Silica gel is used as stationary phase and the products are eluted with a mixture of CHCl₃ : MeOH 40:1 (v/v). The column chromatography is repeated several times with different mixtures of CHCl₃ : MeOH (v/v) to finally obtain pure compounds. The desired compound is chosen, after mass spectrometer and NMR analysis of each obtained compound. *Yield.* 6%

**¹H-NMR** (500 MHz, CDCl₃, 303.57 K, δ ):
9.07 (s, 1H, indolyl-7-H), 9.06 (m, 6H, pyridyl-3,5-H), 9.04 (m, 2H, β-pyrrole-H), 8.87 (m, 4H, β-pyn-ole-H), 8.82 (d, J = 3.93, 2H, β-pyrrole-H), 8.27 (s, 1H, indolyl-2-H), 8.16 (bs, 6H, pyridyl-3,5-H), 7.39 (d, J = 8.40, 1H, indolyl-5-H), 7.15 (d, J = 8.40, 1H, indolyl-4-H), 3.23 (q, J = 7.22, 2H, CH₂-propionyl), 1.51 (t, J = 7.22, 2H, CH₃-propionyl), -2.79 (s, 2H, pyrrole-NH)

**¹³C-NMR** (125 MHz, CDCl₃, 303.57 K, δ ):
172.6 (s, 1C, quart CO-propionyl), 161.0 (s, 4C, quart. □-pyrrole-C), 157.4 (s, 4C, quart. α-pyrrole-C), 150.0 (s, 3C, quart. ipso pyridyl-1-C), 150.0 (s, 1C, quart. ipso indolyl-3-C), 148.5 (d, 6C, pyridyl-3,5-CH), 135.6 (s, 1C, quart. indolyl-8a-C), 134.3 (s, 1C, quart. indolyl-3a-C), 131.3 (d, 2C, β-pyrrole-CH), 131.2 (d, 4C, β-pyrrole-CH), 131.1 (d, 2C, β-pyrrole-CH), 129.5 (d, 6C, pyridyl-2,6-CH), 128.6 (d, 1C, indolyl-2-CH), 128.0 (d, 1C, indolyl-5-CH), 123.5 (s, 1C, quart. meso indolyl-C), 122.0 (d, 1C, indolyl-4-CH), 119.9 (d, 1C, indolyl-7-CH), 118.0 (s, 1C, quart. meso pyridyl-C), 117.8 (s, 2C, quart. meso pyridyl-C), 110.4 (s, 1C, quart. indolyl-6-C), 29.8 (t, 1C, CH₂-propionyl), 8.9 (q, 1C, CH₃-propionyl)

**ESI-MS** (Chloroform/Methanol 1:1, positive ion mode): m/z = 793.19 ([M+H]⁺) C₄₆H₃₁BrN₈O theoretical mass = 790.18, 792.18, 791.18

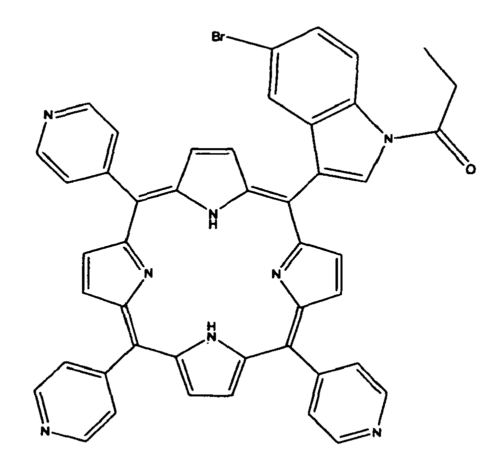

### Example 20

### Synthesis of 5,10,15-tris(N-methylpyridinium-4-yl)-20-(3-(6-bromo-indolyl))-21H,23H-porphyrin chloride ("TMPy₃IndolBr₁P-Cl₃")

To a mixture of 20 ml CH₃NO₂ and 20 ml CHCl₃ (1:1 v/v) 4.3 µmol TPy₃IndolBrN-Prop₁P, and 0.34 mmol methyl p-toluene-sulfonate are added. This reaction mixture is refluxed for 48 h under argon atmosphere. The progress of the N-methylation reaction can be monitored using silica gel TLC and an eluent containing ACN : H₂O(sat. KNO₃) : H₂O 8:1:1 (v/v). Then the reaction mixture is evaporated to dryness, dissolved in deionized water and the impurities are extracted with CHCl₃. The remaining water fraction is filtered and then evaporated to dryness, which gives the tosylate salt of TMPy₃IndolBr₁P³⁺.

TMPy₃IndolBr₁P-tos₃ is dissolved in 35 ml H₂O and 5 ml MeOH. Then 2 g of Dowex 1 x8 Cl⁻ ion exchange resin are added. After 10 h of slow stirring the ion exchange resin is filtered off and the remaining reaction mixture is evaporated to dryness, which gives the Cl⁻ salt of TMPt₃IndolBr₁P³⁺. Yield: 98 %

**¹H-NMR** (500 MHz, CDCl₃, 303.57 K, δ ):
9.38 (bs, 6H, pyridyl-3,5-H), 9.13 (bs, 8H, β-pyrrole-H), 8.95 (bs, 6H, pyridyl-2,6-H), 8.95 (s, 1H, indolyl-NH), 8.19 (s, 1H, indolyl-2-H), 7.98 (s, 1H, indolyl-7-H), 7.30 (bs, 1H, indolyl-5-H), 7.19 (bs, 1H, indolyl-4-H), 4.82 (s, 9H, N⁺-CH₃), -3.66 (s, 2H, pyrrole-NH)

**¹³C-NMR** (125 MHz, CDCl₃, 303.57 K, δ ):
159.8 (s, 3C, quart. ipso pyridyl-1-C), 159.8 (s, 1C, quart. ipso indolyl-3-C), 149.6 (s, 8C, quart. α-pyrrole-C), 145.5 (d, 6C, pyridyl-3,5-CH), 138.0 (s, 1C, quart. indolyl-8a-C), 134.3 (d, 8C, β-pyrrole-CH), 134.0 (d, 6C, pyridyl-2,6-CH), 133.0 (s, 1C, quart indolyl-3a-C), 132.5 (d, 1C, indolyl-2-CH), 124.8 (d, 1C, indolyl-4-CH), 121.5 (d, 1C, indolyl-5-CH), 118.6 (s, 1C, quart. meso indolyl-C), 117.6 (s, 1C, quart. indolyl-6-C), 115.9 (d, 1C, indolyl-7-CH), 115.1 (s, 3C, quart. meso pyridyl-1-C), 48.9 (q, 3C, N⁺-CH₃)

**ESI-MS** (Methanol 1:1, positive ion mode): m/z = 260.73 ([M]³⁺), 390.47 ([M]²⁺)
C₄₆H₃₆BrN₈³⁺ theoretical mass = 779.22, 781.22, 780.22

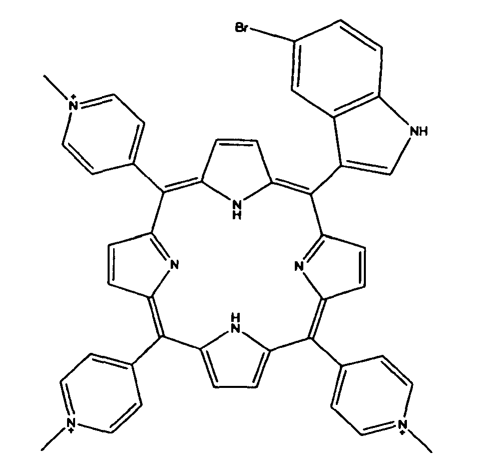

### Mono-substituted meso-(N-methylpyridinium-3-yl) porphyrins modified with 1-pyrene

### Example 21

### Synthesis of 5-(5-bromo-3-pyridyl)-10,15,20-tris(4-pyridyl)-21H,23H-porphyrin ("TPy3PyBr1P")

To a refluxing mixture of 50 ml propionic acid and 10 ml propionic anhydride, 1.5 mmol 5-bromo-3-pyridinecarboxaldehyde, 4.5 mmol 4-pyridinecarboxaldehyde and 6.2 mmol of freshly distilled pyrrole are added. This mixture is refluxed for 150 min. Then the reaction mixture is cooled to room temperature and 2 mmol of 2,3-dichloro-5,6-dicyano-1,4-benzoquinone (DDQ) are added. The mixture is stirred for 1 h, then the solvent is evaporated to dryness and the black-purple product is dissolved in CHCl₃. This mixture is divided into several parts and neutralized with a saturated NaHCO₃ water solution. The neutralized solution is dried over MgSO₄, filtered and evaporated to dryness. Due to the fact that during this statistical synthesis several porphyrin products are obtained, intensive column chromatography is needed to separate the different porphyrin species. Silica gel is used as stationary phase and the products are eluted with a mixture of CHCl₃: MeOH 19:1 (v/v). The column chromatography is repeated several times with different mixtures of CHCl₃ : MeOH v/v to finally obtain pure compounds. The desired compound is chosen, after mass spectrometer and NMR analysis of each obtained compound. Yield: 6%

**¹H-NMR** (500 MHz, CDCl₃, 303.57 K, δ ):
9.37 (d, J = 1.88, 1H, pyridyl-4-α-H), 9.15 (d, J = 1.88, 1H, pyridyl-6-α-H), 9.07 (d, J = 5.35, 6H, pyridyl-3,5-b-H), 8.88 (m, 8H, β-pyrrole-H), 8.69 (m, 1H, pyridyl-2-α-H), 8.17 (d, J = 5.35, 6H, pyridyl-2,6-b-H), -2.93 (s, 2H, pyrrole-NH)

**¹³C-NMR** (125 MHz, CDCl₃, 303.57 K, δ ):
151.8 (d, 1C, pyridyl-4-a-CH), 150.7 (d, 1C, pyridyl-6-a-CH), 149.8 (s, 3C, ipso pyridyl-1-b-C), 148.6 (d, 6C, pyridyl-3,5-b-CH), 146.8 (s, 8C, quart. α-pyrrole-C), 143.3 (d, 1C, pyridyl-2-a-CH), 139.1 (s, 1C, quart. ipso pyridyl-1-a-C), 131.5 (d, 8C, β-pyrrole-CH), 129.5 (d, 6C, pyridyl-2,6-b-CH), 120.0 (s, 1C, quart. meso pyridyl-a-C), 118.1 (s, 3C, quart. meso pyridyl-b-C), 115.5 (s, 1C, quart. pyridyl-5-a-C)

**ESI-MS** (Chloroform/Methanol 1:1, positive ion mode): m/z = 697.53 ([M+H]⁺), 699.53 ([M+H]⁺), 698.53 ([M+H]⁺)
C₄₀H₂₅BrN₈ theoretical mass = 696.14, 698.14, 697.14

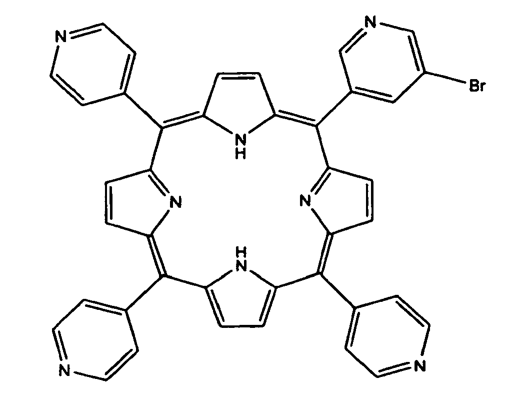

### Example 22

### Synthesis of 5-[(1-pyrenyl)-3-pyridyl]-10,15,20-tris-(4-pyridyl)-21H,23H-porphyrin ("TPy3PyPyr1P")

16.99 µmol TPy₃PyBr₁P and 33.97 µmol pyrene-1-boronic acid are dissolved in 40 ml toluene and 10 ml methanol. Then the mixture is degassed and saturated with argon. After that 0.9 µmol tetrakis(triphenylphosphine)palladium(0) and 1 ml 2 M Na₂CO₃ solution are added under argon atmosphere. Then the reaction mixture is protected from light, heavily stirred and heated up to 80 °C for 120 h. After that, the reaction mixture is filtered, extracted with deionized water, dried over Na₂SO₄ and evaporated to dryness. For the purification of the crude product, intensive column chromatography is needed. Silica gel is used as stationary phase and the product is eluted using a mixture of CHCl₃ :MeOH 49:1 (v/v). The second band is the desired product, which is evaporated to dryness after the separation process. Yield: 94 %

**¹H-NMR** (500 MHz, CDCl₃, 303.57 K, δ ):
9.59 (s, 1H, pyridyl-4-H-a), 9.38 (s, 1H, pyridyl-6-H-a), 9.11 (d, J = 4.19, 2H, β -pyrrole-H), 9.08 (m, 4H, pyridyl-3,5-H-b), 9.08 (m, 2H, pyridyl-3,5-H-c), 8.95 (d, J = 4.19, 2H, β-pyrrole-H), 8.88 (s, 4H, β-pyrrole-H), 8.88 (m, 1H, pyrenyl-H), 8.83 (bs, 1H, Pyridyl-2-H-a), 8.52 (d, J = 9.24, 1H, pyrenyl-H), 8.35 (d, J = 7.91, 1H, pyrenyl-H), 8.30 (d, J = 7.91, 1H, pyrenyl-H), 8.25 (d, J = 8.54, 1H, pyrenyl-H), 8.22 (d, J = 9.29, 2H, pyrenyl-H), 8.18 (M, 4H, pyridyl-2,6-H-b), 8.18 (m, 2H, pyridyl-2,6-H-c), 8.05 (t, J = 7.61, 1H, pyrenyl-H), 7.72 (d, J = 4.56, 1H, pyrenyl-H), -2.84 (s, 2H, pyrrole-NH)

**¹³C-NMR** (125 MHz, CDCl₃, 303.57 K, δ ):
152.3 (d, 1C, pyridyl-4-a-CH), 150.8 (d, 1C, pyridyl-6-a-CH), 150.8 (d, 1C, pyrenyl-CH), 150.1 (s, 4C, quart. ipso pyridyl-l-C), 148.5 (d, 4C, pyridyl-3,5-b-CH); 148.5 (d, 2C, pyridyl-3,5-c-CH), 142.9 (d, 1C, pyridyl-2-a-CH), 137.4 (s, 1C, quart. pyrenyl-C), 135.6 (s, 1C, quart pyrenyl-C), 133.8 (s, 8C, quart. α -pyrrole-C), 132.9 (s, 1C, quart. pyridyl-5-C), 131.7 (s, 2C, quart. pyrenyl), 131.4 (d, 8C, β-pyrrole-CH), 131.0 (s, 1C, quart. pyrenyl-C), 129.2 (s, 1C, quart. pyrenyl-C), 128.1 (d, 1C, pyrenyl-CH), 126.5 (d, 1C, pyrenyl-CH), 125.9 (d, 1C, pyrenyl-CH), 125.5 (d, 2C, pyrenyl-CH), 125.1 (d, 1C, pyrenyl-CH), 125.0 (s, 1C, quart. pyrene-1-C), 124.3 (d, 1C, pyrenyl-CH), 122.3 (d, 1C, pyrenyl-CH), 118.0 (s, 2C, quart. meso pyridyl-1-b-C), 117.8 (s, 1C, quart. meso pyridyl-c-C), 117.0 (s, 1C, meso pyridyl-1-a-C)

**ESI-MS** (Chloroform/Methanol 1:1, positive ion mode): m/z = 819.67 ([M+H]⁺). C₅₆H₃₄N₈ theoretical mass = 818.29

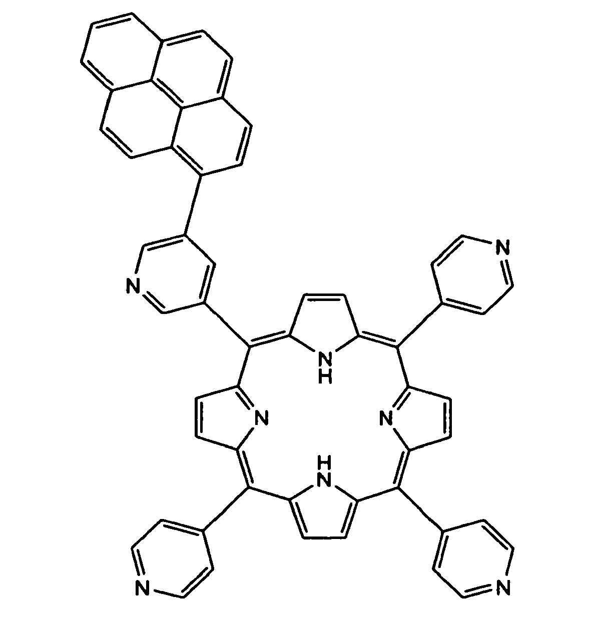

### Example 23

### Synthesis of 5-((1-pyrenyl)-N-methylpyridinium-3-yl)-10,15,20-tris-(N-methyl-pyridinium-4-yl)-21H,23H-porphyrin chloride ("TMPy₃PyPyr₁P-Cl₃")

To a mixture of 5 ml CH₃NO₂ and 5 ml CHCl₃ (1:1 v/v) 11.78 µmol TPy₃PyPyr₁P (7) and 0.943 mmol methyl p-toluene-sulfonate are added. This reaction mixture is refluxed for 161 h under argon atmosphere. The progress of the N-methylation reaction can be monitored using silica gel TLC and an eluent containing ACN : H₂O (sat. KNO₃) : H₂O 8:1:1 (v/v). Then the reaction mixture is evaporated to dryness and dissolved in deionized water and the impurities are extracted with CHCl₃. The remaining water fraction is filtered and then evaporated to dryness, which gives the tosylate salt of TMPy₃PyPyr₁P⁴⁺. TMPy₃PyPyr₁P-tos₄ is dissolved in 35 ml H₂O and 5 ml MeOH and then 2 g of Dowex 1x8 Cl⁻ ion exchange resin are added. After 10 h of slow stirring the ion exchange resin is filtered off and the remaining reaction mixture is evaporated to dryness, which gives the Cl⁻ salt of TMPy₃PyPyr₁P⁴⁺. Yield: 97 %

**¹H-NMR** (500 MHz, CD₃OD, 303.57 K, δ ):
10.18 (s, 1H, pyridyl-4-H-a), 9.88 (s, 1H, pyridyl-6-H-a), 9.68 (s, 1H, pyridyl-2-Ha), 9.53 (bs, 2H, β-pyrrole-H), 9.47 (bs, 6H, pyridyl-3,5-H-b), 9.27 (bs, 2H, β -pyrrole-H), 9.20 (s, 4H, β-pyrrole-H), 9.04 (bs, 6H, pyridyl-2,6-H-b), 8.66 (bs, 1H, pyrenyl-H), 8.52 (bs, 1H, pyrenyl-H), 8.47 (bs, 1H, pyrenyl-H), 8.33 (bs, 1H, pyrenyl-H), 8.24 (bs, 2H, pyrenyl-H), 8.17 (d, J = 8.20, 1H, pyrenyl-H), 8.03 (d, J = 7.20, 1H, pyrenyl-H), 7.71 (d, J = 8.20, 1H, pyrenyl-H), 5.01 (s, 3H, N⁺-CH₃-a), 4.84 (s, 6H, N⁺-CH₃-b), -2.05 (s, 2H, pyrrole-NH)

**¹³C-NMR** (125 MHz, CD₃OD, 303.57 K, δ ):
176.9 (d, 1C, pyridyl-4-CH-a), 158.2 (s, 4C, quart. ipso-pyridyl-1-C), 151.2 (d, 1C, pyridyl-6-a-CH), 147.9 (s, 8C, quart. α-pyrrole-C), 147.8 (d, 1C, pyridyl-2-a-CH), 146.1 (d, 4C, β-pyrrole-CH), 145.8 (d, 4C, β-pyrrole-CH), 145.8 (d, 6C, pyridyl-3,5-b-CH), 141.6 (s, 1C, quart. pyrenyl-C), 134.4 (d, 6C, Pyridyl-2,6-b-CH), 134.2 (d, 1C, pyrenyl-CH), 132.6 (s, 1C, quart. pyridyl-5-a-C), 132.0 (s, 2C, quart. pyrenyl-C), 130.8 (d, 1C, pyrenyl-CH), 130.2 (d, 1C, quart. pyrenyl-C), 129.5 (s, 1C, pyrenyl-CH), 129.2 (s, 1C, quart. pyrenyl-C), 128.2 (d, 1C, pyrenyl-CH), 127.7 (d, 1C, pyrenyl-CH), 126.9 (d, 2C, pyrenyl-CH), 126.4 (d, 1C, pyrenyl-CH), 125.4 (s, 1C, quart. pyrenyl-1-C), 124.4 (d, 1C, pyrenyl-CH), 117.3 (s, 3C, quart. meso pyridyl-a-C), 117.2 (s, 1C, quart. meso pyridyl-b-C), 49.6 (q, 1C, N⁺-CH₃-a), 49.1 (q, 1C, N⁺-CH₃-b)

**ESI-MS** (Chloroform/Methanol 1:1, positive ion mode): m/z = 219.73 ([M⁴⁺]), 292.67 ([M^{3+]}),438.33 ([M²⁺])
C₆₀H₄₆N₈⁴⁺ theoretical mass = 878.38

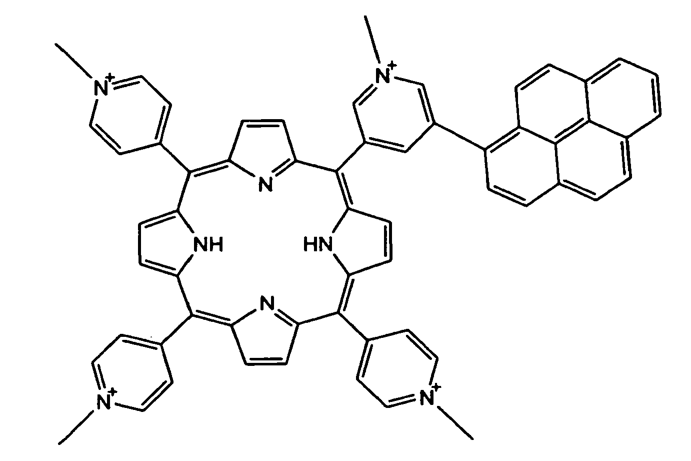

### Example 24

### Synthesis of Gold(III)-5-(5-(2-(9,9-dimethylfluorenyl)-N-methylpyridinium-3-yl))-10,15,20-tris(N-methyl-pyridinium-4-yl)-porphyrin chloride ("Au(III)-TMPy₃PyFluorenyl₁P-Cl₅")

5 µmol TMPy₃PyFluorenyl₁P-Cl₄ and 31 mg LiCl are dissolved in 10 ml H₂O and 250 µl pyridine. Then 22 µmol KAuCl₄ are added, and the reaction mixture is kept at 80 °C for 4 h under argon atmosphere. The progress of the reaction can be monitored by observing the shift of the Soret band of the porphyrin using UV-Vis spectroscopy. After the reaction is complete, the mixture is filtered, evaporated to dryness and the excess of KAuCl₄ is extracted with acetone. The solid dissolved with methanol and gives Au(III)-TMPy₃Pyr₁P-Cl₄ after evaporation of the solvent. Yield: 93 %

**¹H-NMR** (500 MHz, CD₃OD, 303.57 K, δ ):
10.08 (s, 1H,pyridyl-4-a-H), 10.00 (s, 1H, pyridyl-6-a-H), 9.86 (s, 1H, pyridyl-2-aH), 9.86 (m, 2H, β-pyrrole-H), 9.68 (m, 6H, β-pyrrole-H), 9.56 (d, J = 5.40, 6H, pyridyl-3,5-b-H), 9.08 (d, J = 5.40, 6H, pyridyl-2,6-b-H), 8.34 (m, 1H, fluorenyl-H), 8.12 (m, 1H, fluorenyl-H), 8.01 (s, 1H, fluorenyl-H), 7.83 (m, 1H, fluorenyl-H), 7.49 (m, 1H, fluorenyl-H), 7.36 (m, 2H, fluorenyl-H), 4.94 (s, 3H, N⁺-CH₃-a), 4.90 (s, 9H, N⁺-CH₃-b), 1.51 (s, 6H, fluorenyl-CH₃)

**¹³C-NMR** (125 MHz, CD₃OD, 303.57 K, δ ):
159.2 (s, 1C, quart. ipso pyridyl-1-a-C), 159.2 (s, 3C, quart. ipso pyridyl-1-b-C), 156.2 (s, 1C, quart. fluorenyl-9a-C), 155.6 (s, 1C, quart. fluorenyl-8a-C), 146.5 (d, 1C, pyridyl-4-a-CH), 146.5 (d, 1C, pyridyl-6-a-CH), 146.2 (d, 1C, pyridyl-2-a-CH), 146.2 (d, 6C, pyridyl-3,5-b-CH), 143.1 (s, 1C, quart. fluorenyl-4a-C), 143.1 (s, 1C, quart. fluorenyl-4b-C), 139.0 (s, 1C, quart pyridyl-5-a-C), 137.6 (s, 8C, α -pyrrole-C), 135.3 (s, 2C, β-pyrrole-CH), 134.4 (d, 6C, β-pyrrole-CH), 134.4 (d, 6C, pyridyl-2,6-b-CH), 133.0 (s, 1C, quart. ipso-fluorenyl-2-C), 128.5 (d, 2C, fluorenyl-CH), 128.4 (d, 1C, fluorenyl-CH), 123.8 (d, 1C, fluorenyl-CH), 123.8 (d, 1C, fluorenyl-CH), 122.3 (d, 1C, fluorenyl-CH), 121.8 (d, 1C, fluorenyl-CH), 119.6 (s, 1C, quart. meso pyridyl-a-C), 119.6 (s, 3C, quart. meso pyridyl-b-C), 49.8 (q, 1C, N⁺-CH₃-a), 49.5 (q, 3C, N⁺-CH₃-b), 47.9 (s, 1C, quart. fluorenyl-9-C), 27.4 (q, 2C, fluorenyl-CH₃)

**ESI-MS** (Methanol, positive ion mode): m/z = 355.00 ([M]³⁺), 267.07 ([M]⁴⁺), C₅₉H₄₈AuN₈⁵⁺ theoretical mass = 1065.36

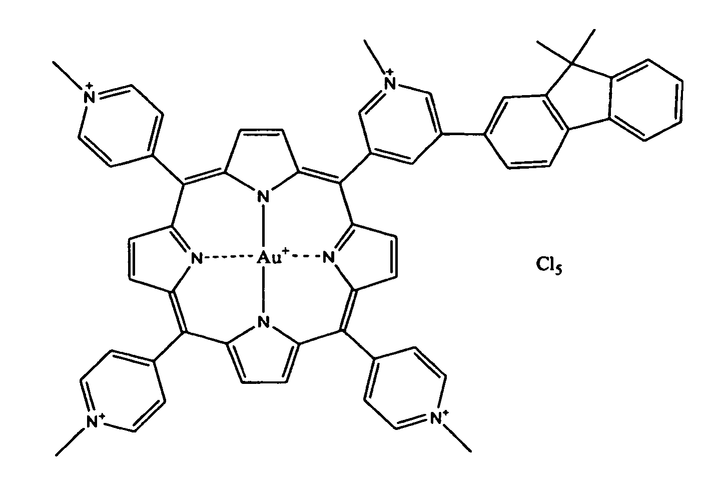

### Example 25

### Synthesis of Gold(III)-5,10,15-tris(N-methylpyridinium-4-yl)-20-(1-pyrenyl)-porphyrin chloride ("Au(III)-TMPy₃Pyr₁P-Cl₄")

6 µmol TMPy₃Pyr₁P-Cl₃ and 40 mg LiCl are dissolved in 10 ml H₂O and 300 µl Pyridine. Then 30 µmol KAuCl₄ are added, and the reaction mixture is kept at 80 °C for 4 h under argon atmosphere. The progress of the reaction can be monitored by observing the shift of the Soret band of the porphyrin using UV-Vis spectroscopy. After the reaction is complete, the mixture is filtered, evaporated to dryness and the excess of KAuCl₄ is extracted with acetone. The solid is dissolved with methanol and gives Au(III)-TMPy₃Pyr₁P-Cl₄ after evaporation of the solvent. Yield: 95 %

**¹H-NMR** (500 MHz, CD₃OD, 303.57 K, δ ):
9.62 (m, 4H, β-pyrrole-H), 9.56 (bs, 2H, pyridyl-3,5-H), 9.46 (bs, 4H, pyridyl-3,5-H), 9.42 (bs, 2H, pyridyl-3,5-H), 9.06 (bs, 2H, pyridyl-2,6-H), 9.00 (bs, 2H, pyridyl-2,6-H), 8.97 (m, 4H, β-pyrrole-H), 8.78 (m, 1H, pyrenyl-H), 8.60 (bs, 1H, pyrenyl-H), 8.31 (bs, 1H, pyrenyl-H), 8.21 (bs, 1H, pyrenyl-H), 8.15 (bs, 1H, pyrenyl-H), 7.79 (m, 2H, pyrenyl-H), 7.44 (bs, 1H, pyrenyl-H), 7.09 (bs, 1H, pyrenyl-H), 4.89 (s, 3H, N⁺-CH₃), 4.81 (s, 6H, N⁺-CH₃)

**¹³C-NMR** (125 MHz, CD₃OD, 303.57 K, δ [ppm]):
156.3 (s, 1C, quart. ipso pyrenyl-1-C), 156.2 (3C, quart. ipso pyridyl-1-C), 146.9 (4C, pyridyl-3,5-a,c-CH), 146.4 (d, 2C, pyridyl-3,5-b-CH), 139.9 (s, 2C, quart α -pyrrole-C), 137.3 (s, 2C, quart α-pyrrole-C), 137.1 (s, 2C, quart α-pyrrole-C), 137.0 (s, 2C, quart α-pyrrole-C), 135.0 (d, 2C, pyridyl-2,6-a-CH), 134.6 (s, 1C, quart. pyrenyl-C), 134.3 (d, 4C, β-pyrrole-CH), 134.1 (d, 4C, β-pyrrole-CH), 133.9 (d, 2C, pyridyl-2,6-c-CH), 133.7 (d, 2C, pyridyl-2,6-b-CH), 133.7 (d, 1C, pyrenyl-CH), 133.4 (s, 1C, quart. pyrenyl-C), 132.6 (s, 1C, quart. pyrenyl-C), 131.3 (s, 1C, quart. pyrenyl-C), 130.8 (d, 1C, pyrenyl-CH), 130.5 (d, 1C, pyrenyl-CH), 129.6 (d, 1C, pyrenyl-CH), 127.6 (d, 2C, pyrenyl-CH), 127.6 (d, 1C, pyrenyl-CH), 126.9 (d, 1C, pyridyl-CH), 125.1 (s, 1C, quart. pyrenyl-C), 124.9 (s, 1C, quart. pyrenyl-C), 124.8 (s, 1C, quart. meso-pyrenyl-C), 124.6 (d, 1C, pyrenyl-CH), 119.1 (s, 2C, meso pyridyl-a,c-C), 118.8 (s, 1C, meso pyridyl-b-C), 49.9 (q, 3C, N⁺-CH₃)

**ESI-MS** (Methanol, positive ion mode): m/z = 245.32 ([M]⁴⁺)
C₅₄H₃₈AuN₇⁴⁺ theoretical mass = 981.28

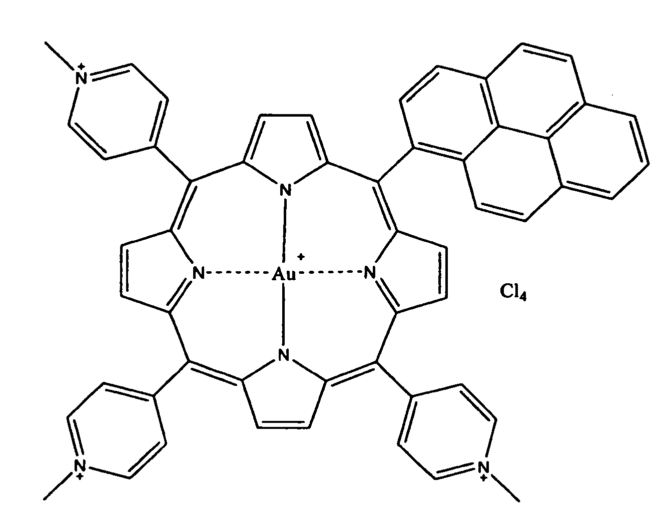

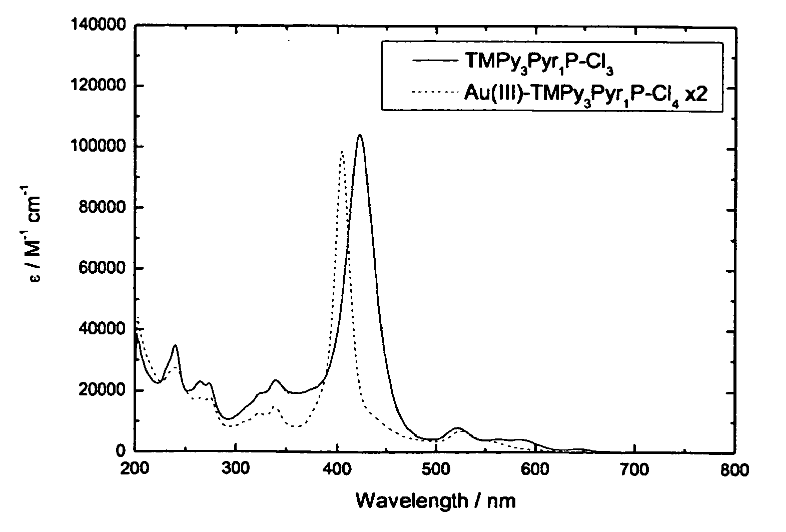

UV-Vis spectra of TMPy₃Pyr₁P-Cl₃, TEPy₃Pyr₁P-Cl₃ and Au(III)-TMPy₃Pyr₁P-Cl₄ in 5 mM (CH₃)₂AsO₂Na, 20 mM NaCl, pH 7.0 buffer at 25 °C.

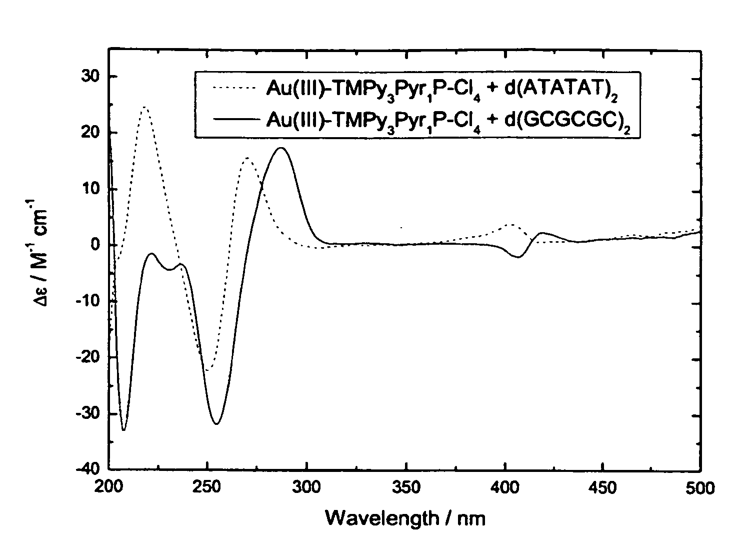

CD-spectra of the complex of Au(III)-TMPy₃Pyr₁P-Cl₄ with d(GCGCGC)₂ (B-DNA) / d(ATATAT)₂ (A-DNA). (0.2 mM DNA, 0.2 mM porphyrin in 5 mM (CH₃)₂AsO₂Na, 20 mM NaCl, pH 7.0 buffer (d(GCGCGC)₂ at 20 °C; d(ATATAT)₂ at 5 °C)

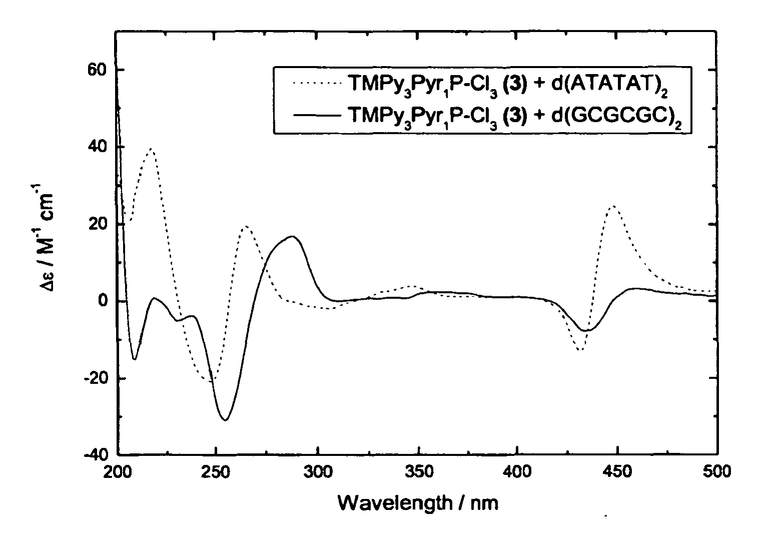

CD-spectra of the complex of TMPy₃Pyr₁P-Cl₃ with d(GCGCGC)₂ (B-DNA) / d(ATATAT)₂ (A-DNA). (0.2 mM DNA, 0.2 mM porphyrin in 5 mM (CH₃)₂AsO₂Na, 20 mM NaCl, pH 7.0 buffer (d(GCGCGC)₂ at 20 °C; d(ATATAT)₂ at 5 °C)

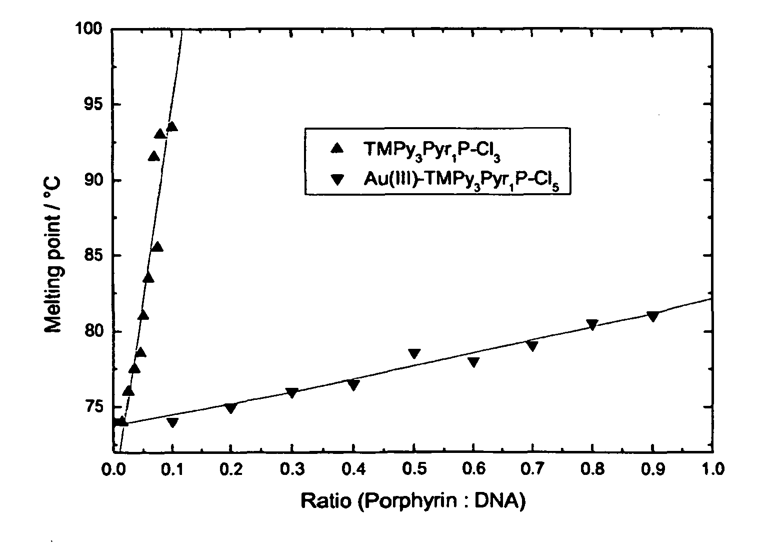

DNA melting points of samples with various porphyrin concentrations in solutions with 0.06 mM CT-DNA in 5 mM (CH₃)₂AsO₂Na, 20 mM NaCl, pH 7.0 buffer.

The figures above show the UV-Vis spectra of the compound Au(III)-TMPy₃Pyr₁P-Cl₄ in comparison to TMPy₃Pyr₁P-Cl₃. These porphyrins show absorption bands > 500 nm and therefore can be used as photosensitizers in photodynamic anticancer therapy (PDT). In the figure below, which depicts the degradation of plasmid DNA by singlet oxygen produced by the porphyrin photosensitizer Au(III)-TMPy₃Pyr₁P-Cl₄, it is proven that this compound can induce photo-oxidative damage to DNA.

Next that that the CT-DNA melting point figures show that these compounds interact with DNA, which can be seen by a drug induced stabilization of the DNA double strand. Circular dichroism spectra show that Au(III)-TMPy₃Pyr₁P-Cl₄ forms porphyrin stacks which bind on the outside of B-DNA while it shows outside binding without any formation of stacks with A-DNA. In comparison to that TMPy₃Pyr₁P-Cl₃ intercalates B-DNA while it forms porphyrin stacks which bind on the outside of A-DNA.

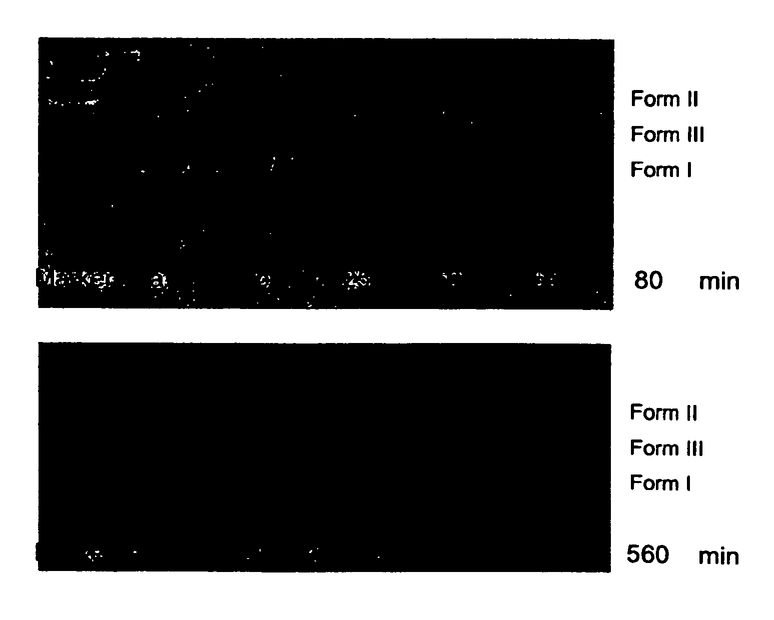

Aggarose gel showing photo-induced unwinding and double strand cleavage of DNA by Au(III)-TMPy₃Pyr₁P-Cl₄. The unwinding of super coiled pBR322 DNA by Au(III)-TMPy₃Pyr₁P-Cl₄ is determined by agarose gel electrophoresis. a) 0 min (without porphyrin) b) 0 min (with porphyrin) irradiation at 420 nm. (0.1 µg/µl pBR322 DNA, 0.32 mM Au(III)-TMPy₃Pyr₁P-Cl₄, 5 mM sodium phosphate, 75 mM NaCl, pH 7.0 buffer).

## Claims

1. Substituted porphyrin compounds of formula 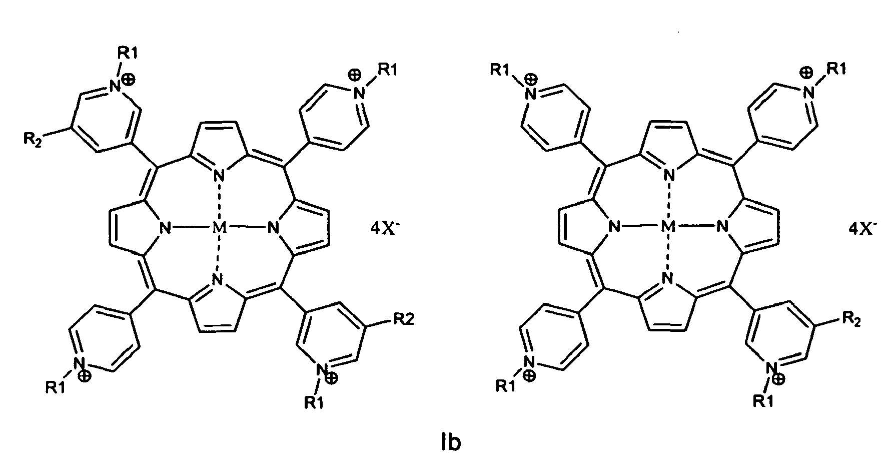 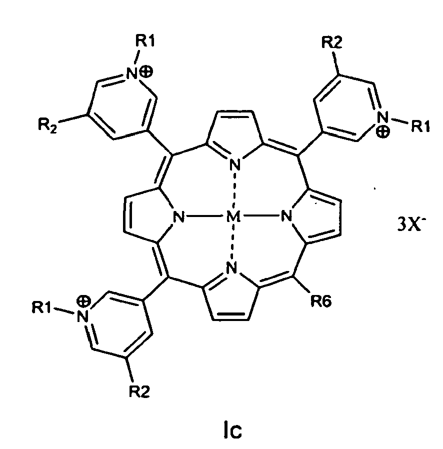 in which R1 is a C₁ to C₄ alkyl group, R2 is halogen or an aryl or heteroaryl group, which may be substituted by groups shown in formula II 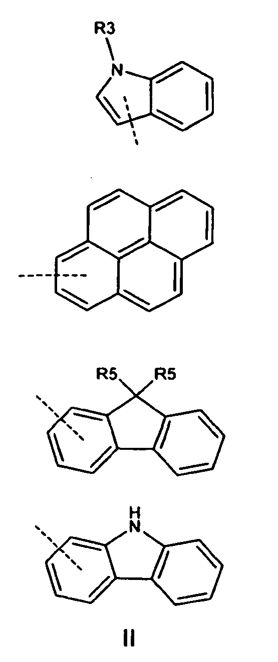 in which R3 is H or a protecting group such as a group SO₂R4, in R4 is a aryl or an heteroaryl group R5 is a H or a C₁ to C₄ alkyl group, R6 are the substituents shown in formula III 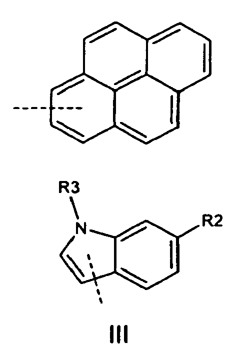 X is a pharmaceutical acceptable counter ion, M is a transition metal ion and pharmaceutically acceptable salts and metal complexes thereof.

2. Substituted porphyrin compound according to claim 1 wherein the compound is Gold(III)-5-(5-(2-(9,9-dimethylfluorenyl)-N-methylpyridinium-3-yl))-10,15,20-tris(N-methyl-pyridinium-4-yl)-porphyrin chloride

3. 5-(5(1-pyrenyl)-N-methylpyridinium-3-yl)-10,15,20-tris-(N-methylpyridinium-4-yl)-21 H,23H-porphyrin chloride

4. 5,10,15-tris(5-(3-(N-phenylsulfonyl-)indolyl)-N-methyl-pyridinium-3-yl)-20-(1-pyrenyl)-21H,23H-porphyrin chloride

5. 5,10-bis(5-(2-(9,9-dimethyl)fluorenyl)-N-methylpyridinium-3-yl)-bis-15,20-(N-methylpyridinium-4-yl)-21 H,23H-porphyrin chloride

6. 5-(5-(2-(9,9-dimethyl)fluorenyl)-N-methylpyridinium-3-yl)-10,15,20-tris(N-methylpyridinium-4-yl)-21 H,23H-porphyrin chloride

7. 5-(5-(2-(9,9-dimethyl)fluorenyl)-N-methylpyridinium-3-yl)-10,15,20-tris(N-methylpyridinium-4-yl)-21H,23H-porphyrin tosylate.

8. Use of the compounds of formula Ib and Ic according to claim 1 for preparing pharmaceutical agents useful in the treatment of cancer

9. Use according to claim 8 wherein the pharmaceutical agent is used in the treatment of medullary thyroid carcinoma.

## Patentansprüche

1. Substituierte Porphyrinverbindungen der Formel 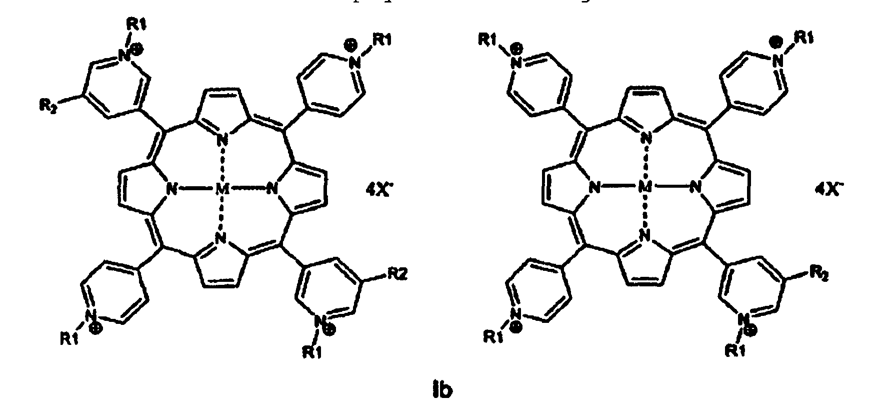 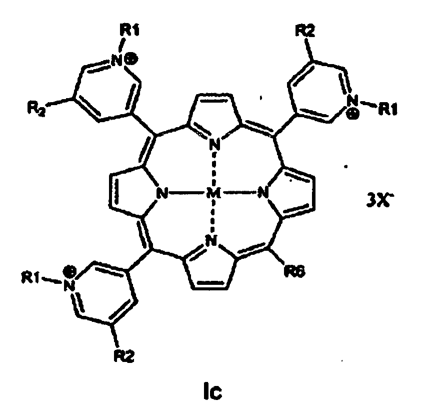 worin R1 für eine C₁- bis C₄-Alkylgruppe steht, R2 für Halogen oder eine Aryl- oder Heteroarylgruppe, die durch in Formel II 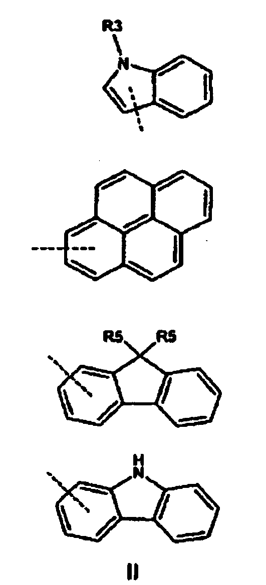 worin R3 H oder eine Schutzgruppe wie eine Gruppe SO₂R4, worin R4 für eine Aryl- oder Heteroarylgruppe steht, bedeutet, R5 H oder eine C₁- bis C₄-Alkylgruppe bedeutet, gezeigte Gruppen substituiert sein kann, steht, R6 für die in Formel III 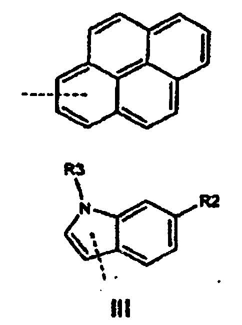 gezeigten Substituenten steht, X für ein pharmazeutisch unbedenkliches Gegenion steht, M für ein Übergangsmetallion steht, und pharmazeutisch unbedenkliche Salze und Metallkomplexe davon.

2. Substituierte Porphyrinverbindung nach Anspruch 1, wobei es sich bei der Verbindung um Gold(III)-5-(5-(2-(9,9-dimethylfluorenyl)-N-methylpyridinium-3-yl))-10,15,20-tris(N-methylpyridinium-4-yl)-porphyrinchlorid

3. 5-(5-(1-Pyrenyl)-N-methylpyridinium-3-yl)-10,15,20-tris(N-methylpyridinium-4-yl)-21H,23H-porphyrinchlorid

4. 5,10,15-Tris(5-(3-(N-Phenylsulfonyl)indolyl)-N-methylpyridinium-3-yl)-20-(1-pyrenyl)-21H,23H-porphyrinchlorid

5. 5,10-Bis(5-(2-(9,9-dimethyl)fluorenyl)-N-methylpyridinium-3-yl)bis-15,20-(N-methylpyridinium-4-yl)-21H,23H-porphyrinchlorid

6. 5-(5-(2-(9,9-Dimethyl)fluorenyl)-N-methylpyridinium-3-yl)-10,15,20-tris(N-methylpyridinium-4-yl)-21H,23H-porphyrinchlorid

7. 5-(5-(2-(9,9-Dimethyl)fluorenyl)-N-methylpyridinium-3-yl)-10,15,20-tris(N-methylpyridinium-4-yl)porphyrintosylat handelt.

8. Verwendung der Verbindungen der Formel Ib oder Ic nach Anspruch 1 zur Herstellung von pharmazeutischen Mitteln, die zur Verwendung bei der Behandlug von Krebs geeignet sind.

9. Verwendung nach Anspruch 3, wobei das pharmazeutische Mittel bei der Behandlung von medullärem Schilddrüsenkarzinom verwendet wird.

## Revendications

1. Composés de porphyrine substituée de formule 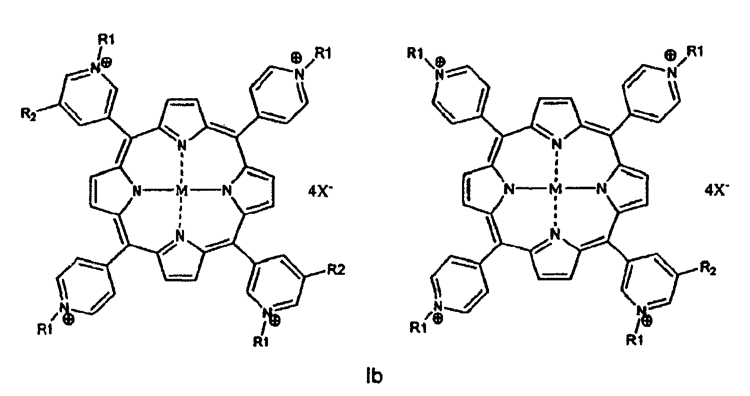 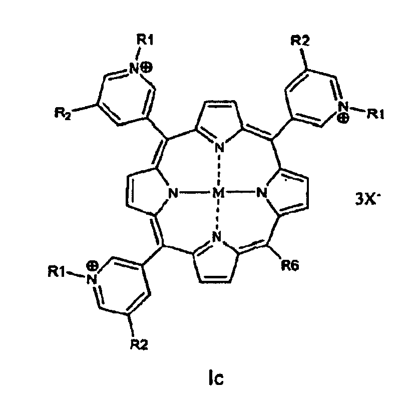 dans lesquels R1 est un groupe alkyle en C₁ à C₄, R2 est un halogène ou un groupe aryle ou hétéroaryle, qui peut être substitué par des groupes montrés dans la formule II 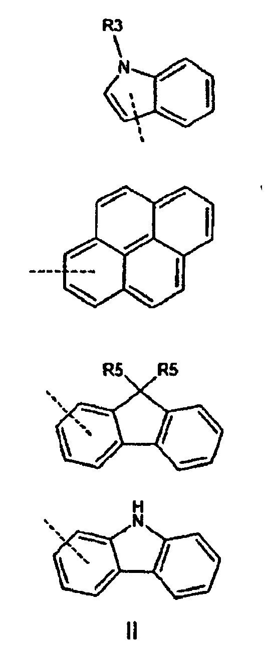 dans laquelle R3 est H ou un groupe protecteur tel qu'un groupe SO₂R4, où R4 est un groupe aryle ou hétéroaryle, R5 est un H ou un groupe alkyle en C₁ à C₄, R6 sont les substituants montrés dans la formule III 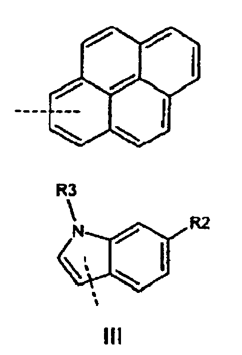 X est un contre-ion pharmaceutiquement acceptable, M est un ion de métal de transition et
leurs seuls et complexes de métaux pharmaceutiquement acceptables.

2. Composés de porphyrine substituée selon la revendication 1, dans lesquels le composé est le chlorure d'or (III)-5-(5-(2-(9,9-diméthylfluorényl)-N-méthylpyridinium-3-yl))-10,15,20-tris(N-méthylpyridinium-4-yl)-porphyrine.

3. Chlorure de 5-(5(1-pyrényl)-N-méthylpyridinium-3-yl)-10,15,20-tris-(N-méthyl-pyridinium-4-yl)-21H,23H-porphyrine.

4. Chlorure de 5,10,15-tris(5-(3-(N-phénylsulfonyl-)indolyl)-N-méthyl-pyridinium-3-yl)-20-(1-pyrényl)-21H,23H-porphyrine.

5. Chlorure de 5,10-bis(5-(2-(9,9-diméthyl)fluorényl)-N-méthylpyridinium-3-yl)-bis-15,20-(N-méthylpyridinium-4-yl)-21H,23H-porphyrine.

6. Chlorure de 5-(5-(2-(9,9-diméthyl)fluorényl)-N-méthylpyridinium-3-yl)-10,15,20-tris(N-méthylpyridinium-4-yl)-21H,23H-porphyrine.

7. Tosylate de 5-(5-(2-(9,9-diméthyl)fluorényl)-N-méthylpyridinium-3-yl)-10,15,20-tris(N-méthylpyridinium-4-yl)-21H,23H-porphyrine.

8. Utilisation des composés de formule Ib et Ic selon la revendication 1, pour préparer des agents pharmaceutiques utiles dans le traitement du cancer.

9. Utilisation selon la revendication 8, dans laquelle l'agent pharmaceutique est utilisé dans le traitement d'un carcinome médullaire de la thyroïde.
